# EUROPEAN PATENT APPLICATION

(11) **EP 2 457 900 A1**
(43) Date of publication of application: **30.05.2012**
(21) Application number: 10382316.7
(22) Date of filing: 25.11.2010
(51) Int. Cl.: C07D 231/12, C07D 401/06, A61K 31/415, A61P 11/06

(54) **New pyrazole derivatives having CRTh2 antagonistic behaviour**

(71) Applicant: Almirall, S.A., 08022 Barcelona (ES)
(72) Inventor: Roberts, Richard Spurring, 08980 Barcelona (ES); Sevilla Gomez, Sara, 08980 Barcelona (ES); Buil Albero, Maria Antonia, 08980 Barcelona (ES)
(74) Representative: Elzaburu Marquez, Alberto

(57) **Abstract**

The present invention relates to a compound of formula (I), to the process for preparing such compounds and to their use in the treatment of a pathological condition or disease susceptible to amelioration by CRTh2 antagonist activity.

## Description

### FIELD OF THE INVENTION

The present invention relates to novel compounds having CRTh2 activity. This invention also relates to pharmaceutical compositions containing them, processes for their preparation and their use in respiratory therapies.

### BACKGROUND OF THE INVENTION

Prostaglandin D2 (PGD2) is a prostaglandin derived from the metabolism of arachidonic acid by cyclooxygenases and downstream PGD2 synthases. In the immune system, PGD2 is mainly produced by mast cells (Lewis et al., J. Immunol., 1982, 129, 1627-1631) but also although at lower levels, by macrophages and Th2 lymphocytes (Urade; J. Immunol, 1989, 143, 2982-2989. Tanaka; J. Immunol, 2000, 164, 2277-2280) in response to local tissue damage and allergic inflammation in diseases such as asthma (Murray et al., N. Engl. J. Med., 1986, 315, 800-804; Liu et al; Am. Rev. Respir. Dis., 1990, 142, 126-132; Wenzel et al., J. Allergy Clin. Immunol., 1991, 87, 540-548), rhinitis (Naclerio et al., Am. Rev. Respir. Dis., 1983, 128, 597-602; Brown et al., Arch. Otolarynol. Head Neck Surg., 1987, 113, 179-183; Lebel et al; J. Allergy Clin. Immunol., 1988, 82, 869-877) and atopic dermatitis among others. Exogenous PGD2 applied to bronchial airways elucidates many characteristics of asthmatic response suggesting that this prostaglandin plays an important pro-inflammatory role in allergic diseases (Hardy; N. Engl. J. Med., 1980, 311, 209-213. Sampson; Thorax, 1997, 52, 513-518). PGD2 binds to three different receptors, the thromboxane-type prostanoid receptor (TP) (Coleman; Br. J. Pharmacol., 1989, 96, 688-692. Francis; Br. J. Pharmacol., 1991, 104, 596-602), the PGD2 receptor (DP also known as DP1) (Boie et al; J. Biol. Chem., 1995, 270, 18910-18916) and the chemoattractant receptor-homologous molecule expressed on Th2 (CRTh2 also known as DP2) (Nagata et al; J. Immunol., 1999, 162, 1278- 1289; Powell; Prostaglandins Leukot. Essent. Fatty Acids, 2003, 69, 179-185). CRTh2 is a Gi-coupling GPCR signalling through reduction of intracellular cAMP and calcium mobilization and it is involved in the chemotaxis of Th2 lymphocytes, eosinophils and basophils (Hirai, J. Exp. Med. 2001, 193, 255-261. Shichijo; J. Pharmacol. Exp. Ther., 2003, 307, 518-525). CRTh2 also inhibits the apoptosis of Th2 lymphocytes (Xue; J. Immunol., 2009, 182, 7580-7586) and stimulates the production of IL4, IL5, and IL13 (Xue; J. Immunol., 2005, 175, 6531-6536) which are cytokines involved in important biological responses such as eosinophil recruitment and survival, mucus secretion, airway hyperresponsiveness and immunoglobulin E production among others. Therefore, molecules that antagonize the mentioned pro-inflammatory PGD2 effects mediated by CRTh2 on key cell types associated with allergic inflammation like basophils, eosinophils and Th2 lymphocytes would have a potential benefit in related pathologies.

In view of these physiological effects, CRTh2 inhibitors of varied chemical structures have been recently disclosed for the treatment or prevention of chronic and acute inflammatory diseases and other pathological conditions, diseases and disorders known to be susceptible to amelioration by inhibition of CRTh2. Several structural families of antagonists are observed. In general antagonists can be grouped into one of the following families: indole acetic acids, phenylacetic acids, phenoxyacetic acids and tetrahydroquinolines derivatives.

Examples of indole acetic acids derivatives are those described in GB2407318, W02003101981, W02005019171, WO2006036994, WO2006092579, WO2007022501, WO2007045867, W02008012511, WO2009063215, US20090192195, US2010063103, WO2009093026, WO2009093029 and GB2457040.

Structurally similar analogues of the indole group have also been reported. Compounds disclosed include structural variations such as: azaindoles (see: GB2422829, GB2422830, GB2422831, WO2005121141, WO2005123731, WO2007065683, WO2007065924, WO2007068418), indolizines (see: WO2006136859, WO2007031747, WO2008074966, WO2008078069, WO2008113965, WO2009044134, WO2009044147), tetrahydoindazoles (see: WO2010006939, WO2010006944), tetrahydroindoles (see: WO2010039982), benzimidazoles (see: WO2006021418, WO2006034418), oxindoles (see: WO2006125784, WO2009061676) and thienopyrroles (see: W02009102462).

Additionally, tricyclic variations of some of the above structures have been disclosed. For examples, see: GB2388540, US2010113503, WO2003097042, WO2005095397, WO2006070325, WO2007019675, WO2008017989, WO2009049021, WO2009140642, WO2010008864, W02010031182, W02010031183, WO2010031184, WO2010054113, WO2010054114, GB 2465062.

For examples of phenylacetic acids and structurally similar analogues, see: US2009197959, WO2004058164, WO2007039736, WO2007052023, WO2007143745, WO2007146838, WO2008024746, WO2008137027, WO2009061730, WO2009085177, WO2009096526, WO2009099901, WO2009102893, W02009108720, WO2009145989, WO2009158426, WO2010003120, WO2010003127, WO2010039977, WO2010040989, W02010042652.

Structurally similar analogues of the phenylacetic group have also been reported. Compounds disclosed include structural variations such as: naphthalene acetic acids (see: WO2010055004, WO2010055005, WO2010055006), pyrimidine acetic acids (see: EP1471057, WO2004096777, WO2005073234, WO2008156780, WO2008156781, WO2009042138, WO2009042139, WO2010027448), pyrrole acetic acids (see: WO2006063763, WO2007144127), thiophene acetic acids (see: WO2005116001, WO2007062677, WO2007062797) and other heterocycle-acetic acids (see: WO2010037054, WO2010037059, W02010057118).

For examples of phenoxyacetic acids see: WO2004089884, WO2004089885, WO2005018529, WO2005105727, WO2005115382, WO2006005909, WO2006021759, WO2006037982, WO2006056752, WO2006125593, WO2006125596, WO2007036743, W02007039741, WO2007062678, WO2007062773, WO2007068894, WO2007144625, WO2008119917, WO2008122784, WO2009004379 , WO2009060209, WO2009089192, WO2010018109, WO2010018112 and W02010018113.

For examples of tetrahydroquinolines see: EP-1413306, EP-1435356, WO2004032848, WO2005007094, WO2005100321, WO2005102338, W02006091674.

Ramatroban, {(3R)-3-[(4-fluorophenyl)sulphonylamino]-1,2,3,4-tetrahydro-9H-carbazole-9-propanoic acid}, a thromboxane A2 receptor antagonist is also known to be a CRTh2 antagonist (Sugimoto et al; J. Pharmacol. Exp. Ther., 2003, 305, 347-352). A few compounds having the capacity to selectively inhibit CRTh2 are in active development. Examples of these compounds are ODC-9101, AZD-1981, ACT-129968, AP-761, AM211, AM461, ADC-3680, ARRY-502 and RG-7185.

In addition, compounds having the capacity to selectively inhibit both DP1 and CRTh2 are in active development. An example of this type of compound is AMG-853.

These observations suggest that CRTh2 antagonists may have valuable properties for the treatment of diseases mediated by PGD2.

### SUMMARY OF THE INVENTION

The invention provides a compound of formula (I), or a pharmaceutically acceptable salt, N-oxide, solvate, stereiosomer or deuterated derivative thereof: 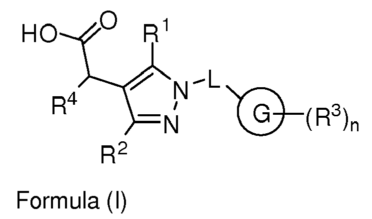 wherein:
- R¹ represents a linear or branched C₁-C₄ alkyl group;
- R² is selected from the group consisting of a linear or branched C₁-C₄ alkyl group, a hydroxyl group, a C₁-C₄ alkoxy group and a C₆₋₁₀ aryl group, wherein the aryl group and the heteroaryl group are optionally substituted by one or more substituents selected from the group consisting of a halogen atom, a C₁-C₄ alkyl group, a C₁-C₄ alkoxy group, a cyano group, a -CF₃ group and a nitro group;
- each R³ is the same or different and is selected from the group consisting of a halogen atom, a cyano group, a carboxy group, a C₆-C₁₀ aryl group, a C₆-C₁₀ aryloxy group, a (C₆-C₁₀ aryl)-(C₁-C₄ alkyl) group, a 3- to 7-membered heterocyclyl group containing at least one heteroatom selected from N, S and O, a (3- to 7-membered heterocyclyl)-(C₁-C₄ alkyl) group, a 5- to 10-membered heteroaryl group containing at least one heteroatom selected from N, S and O, a (5- to 10-membered heteroaryl)-(C₁-C₄ alkyl) group, a -(CH₂)₀₋₁-SO₂R^{a} group, a -O(CH₂)₀₋₁-R^{a} group, a -NHCOR^{a} group, a -CONHR^{a} group, a -NHCONHR^{a} group, a -NHSO₂R^{a} group, a -SO₂N(CH₃)R^{a} group and a -COR^{a} group;
- R⁴ is selected from the group consisting of a hydrogen atom and a linear or branched C₁-C₄ alkyl group;
- R^{a} is selected from the group consisting of a linear or branched C₁-C₄ alkyl group, a C₆-C₁₀ aryl group, a (C₆-C₁₀ aryl)-(C₁-C₄ alkyl) group, a (C₆-C₁₀ aryl)-(C₂-C₆ alkenyl) group, a (C₆-C₁₀ aryl)-(C₃-C₇ cycloalkyl) group, a 3- to 7-membered heterocyclyl group containing at least one heteroatom selected from N, S and O, a 5- to 10-membered heteroaryl group containing at least one heteroatom selected from N, S and O; and a (5- to 10-membered heteroaryl)-(C₁-C₄ alkyl) group, wherein the aryl and heteroaryl groups are optionally substituted by one or more substituents selected from the group consisting of a linear or branched C₁-C₄ alkyl group, a C₁-C₄ alkoxy group, a halogen atom, a - CF₃ group, a -CN group and a -SO₂(C₁-C₄ alkyl) group;
- n has a value of 0 to 3;
- L is selected from the group consisting of a direct bond, a -CR^{b}R^{c}- group and a - SO₂- group;
- R^{b} and R^{c} are independently selected from the group consisting of a hydrogen atom and a linear or branched C₁-C₄ alkyl group; or R^{b} and R^{c} together with the carbon atom to which they are attached form a C₃-C₇ cycloalkyl group;
- G is selected from the group consisting of a phenyl group, a pyridyl group, a quinolinyl group and an isoquinolinyl group;
with the proviso that when G is phenyl or pyridyl group, n can not have a value of 0; with the additional proviso that when L is a direct bond or a methylene group, R³ is other than a halogen atom or a -CN group;
and with the additional proviso that the compound is not 2-(3,5-dimethyl-1-(4-(piperidine-1-carbonyl)phenyl)-1H-pyrazol-4-yl)acetic acid or 2-(1-(biphenyl-4-yl)-3,5-dimethyl-1 H-pyrazol-4-yl)acetic acid.

The invention provides synthetic processes and intermediates described herein, which are useful for preparing compounds of the invention.

The invention also provides a pharmaceutical composition comprising a compound of the invention and a pharmaceutically-acceptable carrier. The invention further provides combinations comprising a compound of the invention and one or more other therapeutic agents and pharmaceutical compositions comprising such combinations.

The invention also provides a compound of the invention for use in the treatment of the human or animal body by therapy.

The invention also provides a compound of the invention for use in the treatment of a disease or condition susceptible to amelioration by CRTh2 antagonists, in a mammal, in particular wherein the pathological condition or disease is selected from asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, irritable bowel disease and inflammatory bowel disease (such as Crohn's disease and ulcerative colitis).

The invention also provides the use of a compound of the invention in the manufacture of a formulation or medicament for treating a disease or condition susceptible to amelioration by CRTh2 antagonists, in particular wherein the condition or disease is as described above.

The invention also provides a method of treating a disease or condition as described above; comprising such method administering to the mammal, a therapeutically effective amount of a compound of the invention. The invention further provides a method of treatment comprising administering a therapeutically effective amount of a combination of a compound of the invention together with one or more other therapeutic agents.

### DETAILED DESCRIPTION OF THE INVENTION

When describing the compounds, compositions, combinations and methods of the invention, the following terms have the following meanings, unless otherwise indicated.

As used herein the term C₁₋₄ alkyl embraces optionally substituted, linear or branched radicals having 1 to 4 carbon atoms. Examples include methyl, ethyl, n-propyl, i-propyl, n-butyl, sec-butyl or t-butyl.

As used herein, the term C₂₋₆ alkenyl embraces optionally substituted, linear or branched, mono or polyunsaturated radicals having 2 to 6 carbon atoms, preferably, 2 to 4 carbon atoms. Examples include vinyl, allyl, 1-propenyl, isopropenyl, 1-butenyl, 2-butenyl and 3-butenyl radicals.

As used herein, the term C₁₋₄ alkoxy (or alkyloxy) embraces optionally substituted, linear or branched oxy-containing radicals each having alkyl portions of 1 to 4 carbon atoms. Examples of C₁₋₄ alkoxy radicals include methoxy, ethoxy, n-propoxy, i-propoxy, n-butoxy, sec-butoxy and t-butoxy.

As used herein, the term C₃₋₇ cycloalkyl group embraces saturated carbocyclic radicals monocyclic or polycyclic ring having from 3 to 7 carbon atoms. Examples include cyclopropyl, cyclobutyl, cyclopentyl, cyclohexyl and cycloheptyl.

As used herein, the term C₆₋₁₀ aryl radical embraces typically a C₆-C₁₀ monocyclic or polycyclic aryl radical such as phenyl and naphthyl.

As used herein, the term C₆₋₁₀ aryloxy embraces oxy-containing radicals each having a C₆-C₁₀ monocyclic or polycyclic aryl moiety. Examples include phenoxy and naphthyloxy, with phenoxy preferred.

As used herein, the term 5- to 10- membered heteroaryl radical embraces typically a 5-to 10- membered ring system comprising at least one heteroaromatic ring and containing at least one heteroatom selected from O, S and N. A 5- to 10- membered heteroaryl radical may be a single ring or two or more fused rings wherein at least one ring contains a heteroatom. Examples include pyridinyl, pyrazinyl, pyrimidinyl, pyridazinyl, furyl, benzofuranyl, oxadiazolyl, oxazolyl, isoxazolyl, benzoxazolyl, benzisoxazolyl, imidazolyl, benzimidazolyl, thiazolyl, thiadiazolyl, isothiazolyl, benzisothiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, 1H-pyrazolo[3,4-d]pyridinyl, thieno[2,3-d] pyrimidinyl and the various pyrrolopyridinyl radicals.

As used herein, the term 3 to 7-membered heterocyclyl radical embraces typically a non-aromatic, saturated or unsaturated C₃-C₇ carbocyclic ring system in which one or more carbon atoms are replaced by a heteroatom selected from N, O and S. Examples of 3 to 7-membered heterocyclic radicals include piperidinyl, pyrrolidinyl, pyrrolinyl, piperazinyl, morpholinyl, thiomorpholinyl, pyrazolinyl, azetidinyl, oxetanyl, tetrahydrofuranyl, tetrahydrothiophenyl, tetrahydropyranyl, tetrahydrothiopyranyl, 1,1-dioxotetrahydrothiopyranyl and dioxanyl.

When R³ represents a (C₆-C₁₀ aryl)-(C₁-C₄ alkyl) group, a (3- to 7-membered heterocycyl)-(C₁-C₄ alkyl) group or a (5- to 10-membered heteroaryl)-(C₁-C₄ alkyl) group, it is to be understood that the (C₁-C₄ alkyl) portion is bonded to the G substituent of the molecule as depicted in formula (I).

When R^{a} represents a (C₆-C₁₀ aryl)-(C₁-C₄ alkyl) group, a (C₆-C₁₀ aryl)-(C₁-C₄ alkenyl) group or a (C₆-C₁₀ aryl)-(C₃-C₇ cycloakyl) group, it is to be understood that the C₆-C₁₀ aryl portion is the terminal radical of the whole R³ substituent.

As used herein, the term halogen atom embraces chlorine, fluorine, bromine or iodine atoms typically a fluorine, chlorine or bromine atom. The term halo when used as a prefix has the same meaning.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition associated with CRTh2 activity" includes all disease states and/or conditions that are acknowledged now, or that are found in the future, to be associated with CRTh2 activity. Such disease states include, but are not limited to, asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, irritable bowel disease and inflammatory bowel disease (such as Crohn's disease and ulcerative colitis). Preferably, such disease states are asthma or chronic obstructive pulmonary disease (COPD), rhinitis and atopic dermatitis.

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, formic, fumaric, gluconic, glutamic, hydrobromic, hydrochloric, hydrofluoric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid), napadisilic (1,5-naphthalenedisulfonic acid), triphenyl acetic and the like. Particularly preferred are salts derived from formic, fumaric, hydrobromic, hydrochloric, hydrofluoric, acetic, sulfuric, methanesulfonic, xinafoic, tartaric, maleic, succinic and napadisilic acids.

Salts derived from pharmaceutically-acceptable inorganic bases include aluminum, calcium, copper, ferric, ferrous, lithium, magnesium, manganic, manganous, potassium, sodium, zinc and the like. Particularly preferred are calcium, magnesium, potassium and sodium salts.

Salts derived from pharmaceutically-acceptable organic bases include salts of ammonia, primary, secondary and tertiary amines, including substituted amines, cyclic amines, naturally-occurring amines and the like, such as ammonia, arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C, are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled compounds include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

The term "amino-protecting group" refers to a protecting group suitable for preventing undesired reactions at amino nitrogen. Representative amino-protecting groups include, but are not limited to, formyl; acyl groups, for example alkanoyl groups such as acetyl, benzoyl, pivaloyl or trifluoroacetyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups such as benzyl (Bn), trityl (Tr), and 1,1-di-(4'-methoxyphenyl)methyl and the like.

The term "hydroxy-protecting group" refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl, pivaloyl or trifluoroacetyl; arylcarbonyl groups, such as benzoyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

The compounds of the invention contain at least a chiral center. Accordingly, the invention includes racemic mixtures, enantiomers, and mixtures enriched in one or more stereoisomer. The scope of the invention as described and claimed encompasses the racemic forms of the compounds as well as the individual enantiomers, diastereomers, and stereoisomer-enriched mixtures.

Typically, R¹ is selected from the group consisting of a linear C₁-C₂ alkyl group; preferably R¹ represents a methyl group.

Typically, R² is selected from the group consisting of a C₁-C₂ alkyl group, a hydroxyl group and a phenyl group, wherein the phenyl group is optionally substituted with a halogen atom. Preferably R² is selected from the group consisting of a methyl group and a phenyl group, wherein the phenyl group is optionally substituted with a chlorine atom.

Typically, R⁴ is selected from the group consisting of a hydrogen atom and a C₁-C₂ alkyl group, preferably R⁴ represents a hydrogen atom.

Typically, L is selected from the group consisting of a -CH₂- group and a -SO₂-group.

Typically G represents a phenyl group.

Typically R³ is selected from the group consisting of a benzyl group, a -(CH₂)₀₋₁-SO₂R^{a} group, a -NHCOR^{a} group and a -NHSO₂R^{a} group. Preferably R³ represents a -SO₂R^{a} group.

Typically n is 0, 1 or 2, preferably 0 or 1.

Typically R^{a} is selected from the group consisting of a C₆-C₁₀ aryl group and a 3- to 7-membered heterocyclyl group, wherein the aryl group is optionally substituted by one or two substituents selected from the group consisting of a linear or branched C₁-C₄ alkyl group, a C₁-C₄ alkoxy group, a halogen atom, a -CF₃ group, a -CN group and a - SO₂(C₁-C₄ alkyl) group. Preferably R^{a} is selected from the group consisting of a phenyl group and a morpholinyl group.

Typically R³ is selected from the group consisting of a benzyl group, a -(CH₂)₀₋₁-SO₂R^{a} group, a -NHCOR^{a} group and a -NHSO₂R^{a} group, wherein R^{a} is selected from the group consisting of a C₆-C₁₀ aryl group, a 3- to 7-membered heterocyclyl group, and wherein the aryl group is optionally substituted by one or two substituents selected from the group consisting of a linear or branched C₁-C₄ alkyl group, a C₁-C₄ alkoxy group, a halogen atom, a -CF₃ group, a -CN group and a -SO₂(C₁-C₄ alkyl) group; and n is 0 or 1. Preferably, R³ represents a -SO₂R^{a} group, wherein R^{a} is selected from the group consisting of a phenyl group and a morpholinyl group; and n has a value of 1.

In a preferred embodiment of the present invention, R¹ represents a methyl group, R² is selected from the group consisting of a methyl group and a phenyl group, wherein the phenyl group is optionally substituted with a chlorine atom, R⁴ represents a hydrogen atom, L is selected from the group consisting of a -CH₂- group and a -SO₂-group, G represents a phenyl group, R³ is selected from the group consisting of a -SO₂R^{a} group, wherein R^{a} is selected from the group consisting of a phenyl group and a morpholinyl group and n has a value of 1.

In another embodiment of the present invention, R¹ is selected from the group consisting of a methyl group; R² is selected from the group consisting of a methyl group, a hydroxyl group and a phenyl group optionally substituted by a chlorine atom; R³ is selected from the group consisting of a chlorine atom, a cyano group, a carboxy group, a phenyl group, a phenoxy group, a benzyl group, a -(CH₂)₀₋₁-SO₂R^{a} group, a-O(CH₂)₀₋₁-R^{a} group, a -NHCOR^{a} group, a -CONHR^{a} group, a -NHCONHR^{a} group, a-NHSO₂R^{a} group, a -SO₂N(CH₃)R^{a} group and a -COR^{a} group; R⁴ represents a hydrogen atom; R^{a} is selected from the group consisting of a methyl group, a phenyl, a benzyl group, a phenyl-(C₂-C₆ alkenyl) group, a phenyl-cyclopropyl group, a piperidin-1-yl group, a morpholinyl group, a piperazin-1-yl group, wherein the phenyl group is optionally substituted by one or two substituents selected from the group consisting of a methyl group, a chlorine atom, a fluorine atom and a -CF₃ group, n has a value of 0, 1 or 2; L is selected from the group consisting of a direct bond, a -CH₂- group and a - SO₂-group; and G is selected from the group consisting of a phenyl group and a quinoline group.

Particular individual compounds of the invention include:
{3,5-dimethyl-1-[3-({[*trans*-2-phenylcyclopropyl]carbonyl}amino)phenyl]-1H-pyrazol-4-yl}acetic acid ;
[3,5-dimethyl-1-(3-{[4-(trifluoromethyl)benzoyl]amino}benzyl)-1H-pyrazol-4-yl]acetic acid ;
[3,5-dimethyl-1-(3-{[(4-methylphenyl)sulfonyl]amino}benzyl)-1H-pyrazol-4-yl]acetic acid;
{3,5-dimethyl-1-[3-({[(4-methylphenyl)amino]carbonyl}amino)benzyl]-1H-pyrazol-4-yl}acetic acid;
(1-{3-[(3-fluorobenzoyl)amino]benzyl}-3,5-dimethyl-1H-pyrazol-4-yl)acetic acid;
[1-(3-{[(2,5-dichlorophenyl)sulfonyl]amino}benzyl)-3,5-dimethyl-1H-pyrazol-4-yl]acetic acid;
[3,5-dimethyl-1-(3-{[(4-methylphenyl)sulfonyl]amino}phenyl)-1H-pyrazol-4-yl]acetic acid;
(1-{3-[(benzylsulfonyl)amino]phenyl}-3,5-dimethyl-1H-pyrazol-4-yl)acetic acid;
{1-[3-({[(4-fluorophenyl)amino]carbonyl}amino)phenyl]-3,5-dimethyl-1H-pyrazol-4-yl}acetic acid;
{1-[(4-cyanophenyl)sulfonyl]-3,5-dimethyl-1H-pyrazol-4-yl}acetic acid;
(1-{4-[(benzylamino)carbonyl]benzyl}-3,5-dimethyl-1H-pyrazol-4-yl)acetic acid;
{1-[(3,4-dichlorophenyl)sulfonyl]-5-methyl-3-phenyl-1H-pyrazol-4-yl}acetic acid;
{3,5-dimethyl-1-[2-(phenylsulfonyl)benzyl]-1H-pyrazol-4-yl}acetic acid;
[5-methyl-3-phenyl-1-(quinolin-2-ylmethyl)-1H-pyrazol-4-yl]acetic acid;
{5-methyl-3-phenyl-1-[2-(phenylsulfonyl)benzyl]-1H-pyrazol-4-yl}acetic acid;
{3,5-dimethyl-1-[4-(methylsulfonyl)benzyl]-1H-pyrazol-4-yl}acetic acid;
(5-methyl-3-phenyl-1-quinolin-4-yl-1H-pyrazol-4-yl)acetic acid;
{3,5-dimethyl-1-[3-({[trans-2-phenylcyclopropyl]amino}carbonyl)phenyl]-1H-pyrazol-4-yl}acetic acid;
[1-(biphenyl-2-ylmethyl)-5-methyl-3-phenyl-1H-pyrazol-4-yl]acetic acid;
(5-methyl-3-phenyl-1-{2-[(phenylsulfonyl)methyl]benzyl}-1H-pyrazol-4-yl)acetic acid;
{5-methyl-3-phenyl-1-[2-(piperidin-1-ylcarbonyl)benzyl]-1H-pyrazol-4-yl}acetic acid;
[5-methyl-1-(2-phenoxybenzyl)-3-phenyl-1H-pyrazol-4-yl]acetic acid;
{5-methyl-1-[2-(morpholin-4-ylsulfonyl)benzyl]-3-phenyl-1H-pyrazol-4-yl}acetic acid;
[1-(2-benzylbenzyl)-5-methyl-3-phenyl-1H-pyrazol-4-yl]acetic acid;
{3-(4-chlorophenyl)-5-methyl-1-[2-(morpholin-4-ylsulfonyl)benzyl]-1H-pyrazol-4-yl}acetic acid;
{3-(4-chlorophenyl)-5-methyl-1-[2-(phenylsulfonyl)benzyl]-1H-pyrazol-4-yl}acetic acid;
(1-{2-[(dimethylamino)sulfonyl]benzyl}-5-methyl-3-phenyl-1H-pyrazol-4-yl)acetic acid;
[1-(2-benzoylbenzyl)-5-methyl-3-phenyl-1H-pyrazol-4-yl]acetic acid;
{3-hydroxy-5-methyl-1-[2-(phenylsulfonyl)benzyl]-1H-pyrazol-4-yl}acetic acid;
{5-methyl-3-phenyl-1-[2-(piperidin-1-ylsulfonyl)benzyl]-1H-pyrazol-4-yl}acetic acid; and
{1-[4-fluoro-2-(piperazin-1-ylsulfonyl)benzyl]-5-methyl-3-phenyl-1H-pyrazol-4-yl}acetic acid,
or a pharmaceutically acceptable salt, N-oxide, solvate, stereiosomer or deuterated derivative thereof.

Of particular interest are the compounds:
{3,5-dimethyl-1-[3-({[*trans*-2-phenylcyclopropyl]carbonyl}amino)phenyl]-1H-pyrazol-4-yl}acetic acid ;
[3,5-dimethyl-1-(3-{[(4-methylphenyl)sulfonyl]amino}benzyl)-1H-pyrazol-4-yl]acetic acid;
{3,5-dimethyl-1-[3-({[(4-methylphenyl)amino]carbonyl}amino)benzyl]-1H-pyrazol-4-yl}acetic acid;
[1-(3-{[(2,5-dichlorophenyl)sulfonyl]amino}benzyl)-3,5-dimethyl-1H-pyrazol-4-yl]acetic acid;
{3,5-dimethyl-1-[2-(phenylsulfonyl)benzyl]-1H-pyrazol-4-yl}acetic acid;
{5-methyl-3-phenyl-1-[2-(phenylsulfonyl)benzyl]-1H-pyrazol-4-yl}acetic acid;
{5-methyl-1-[2-(morpholin-4-ylsulfonyl)benzyl]-3-phenyl-1H-pyrazol-4-yl}acetic acid;
[1-(2-benzylbenzyl)-5-methyl-3-phenyl-1H-pyrazol-4-yl]acetic acid;
{3-(4-chlorophenyl)-5-methyl-1-[2-(morpholin-4-ylsulfonyl)benzyl]-1H-pyrazol-4-yl}acetic acid;
{3-(4-chlorophenyl)-5-methyl-1-[2-(phenylsulfonyl)benzyl]-1H-pyrazol-4-yl}acetic acid;
{3-hydroxy-5-methyl-1-[2-(phenylsulfonyl)benzyl]-1H-pyrazol-4-yl}acetic acid;
{5-methyl-3-phenyl-1-[2-(piperidin-1-ylsulfonyl)benzyl]-1H-pyrazol-4-yl}acetic acid;

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein. Processes for preparing compounds of the invention are provided as further embodiments of the invention and are illustrated by the procedures below. Compounds of general formula (I) may be prepared following the synthetic scheme depicted in Figure 1.

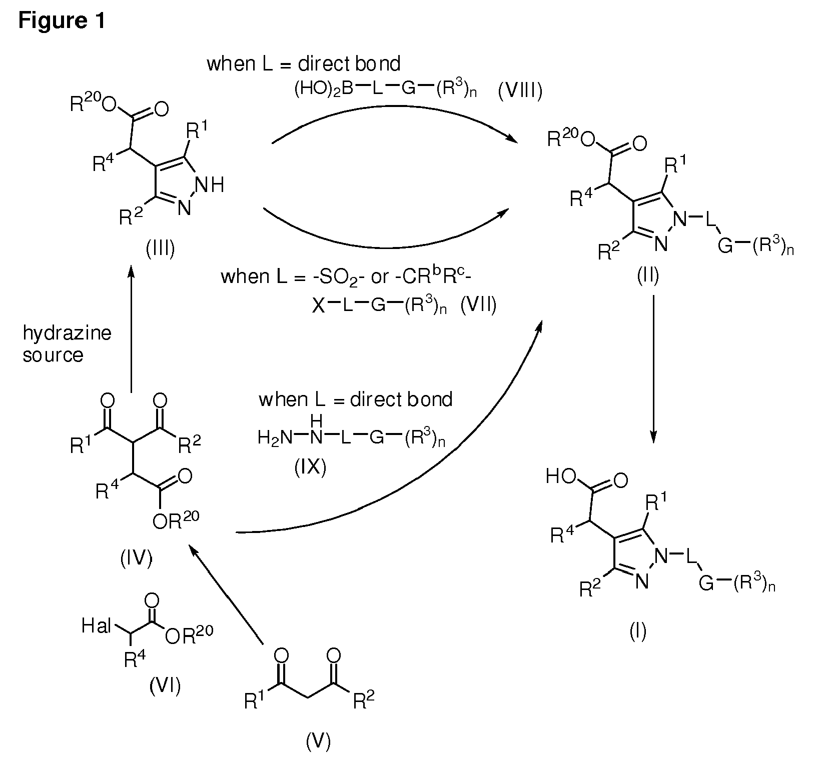

Compounds of general formula (I) may be prepared by hydrolysis of the ester group of a compound of formula (II) wherein R²⁰ represents an alkyl group such as methyl, ethyl, propyl, benzyl or tert-butyl. The reaction is carried out by mixing a compound of formula (II) wherein R²⁰ is defined as above with an acid such as trifluoroacetic acid in a solvent such as dichloromethane or chloroform at a temperature from 0ºC to the boiling point of the solvent to yield the compound of formula (I).

In the particular case where L represents a direct bond, compounds of formula (II) wherein R²⁰ is as described above may be prepared by a coupling reaction between a pyrazole derivative of formula (III) and a boronic acid of formula (VIII) wherein L represents a direct bond. The reaction is carried out in the presence of a transition metal catalyst such as copper acetate and base such as pyridine in a solvent such as dichloromethane under an atmosphere of oxygen at a temperature from ambient to the boiling point of the solvent to yield the compound of formula (II).

Alternatively, in the particular case where L represents a direct bond, compounds of general formula (II) may be prepared by the reaction of a diketone of formula (IV) with a hydrazine of formula (IX) wherein L represents a direct bond. The reaction is carried out in the presence or absence of a base such as triethylamine, in a solvent such as acetic acid at a temperature from ambient to the boiling point of the solvent to yield the compound of formula (II).

In the particular case where L represents a -SO₂- group, compounds of formula (II) may be prepared by the reaction with a sulphonylating agent of formula (VII) wherein L represents a -SO₂- group and X represents a halogen atom such as chlorine, bromine or iodine. The reaction is carried out by mixing a compound of formula (III) with the sulphonylating agent of formula (VII) wherein L and X are defined above in a solvent such as tetrahydrofuran in the presence of a base such as sodium hydride at a temperature from 0ºC to 100ºC to yield the compound of formula (II).

In the particular case where L represents a -CR^{b}R^{c}- group, compounds of formula (II) may be prepared by addition of an alkylating agent of formula (VII) wherein L represents a -CR^{b}R^{c}- group and X represents either a halogen atom such as chlorine, bromine or iodine or a leaving group such as methanesulphonate. The reaction is carried out by mixing the compound of formula (III) with an alkylating agent of formula (VII) wherein L and X are defined above, in a solvent such as dimethylformamide in the presence of a base such as potassium carbonate or sodium hydride to yield the compound of formula (II).

Compounds of general formula (III) may be prepared by the reaction of a diketone of formula (IV) with a hydrazine source. The reaction is carried out by mixing a compound of formula (IV) with a hydrazine source such as hydrazine monohydrate in the presence or absence of a base such as sodium acetate, in a solvent such as acetic acid at a temperature from ambient to 100ºC to yield the compound of formula (III). Compounds of general formula (IV) may be prepared by the reaction of a diketone of formula (V) with an alkylating agent of formula (VI) wherein Hal is a halogen atom such as chlorine, bromine or iodine. The reaction is carried out by mixing a compound of formula (V) with an alkylating agent of formula (VI) in the presence of a base such as sodium hydride in a solvent such as dimethylformamide at a temperature from ambient to 70 ºC to yield the compound of formula (IV).

In the particular case where R³ represents a -NHCOR^{a} group, a -NHCONHR^{a} group or a -NHSO₂R^{a} group and n is 1, compounds of general formula (II) may be prepared following the synthetic scheme depicted in Figure 2.

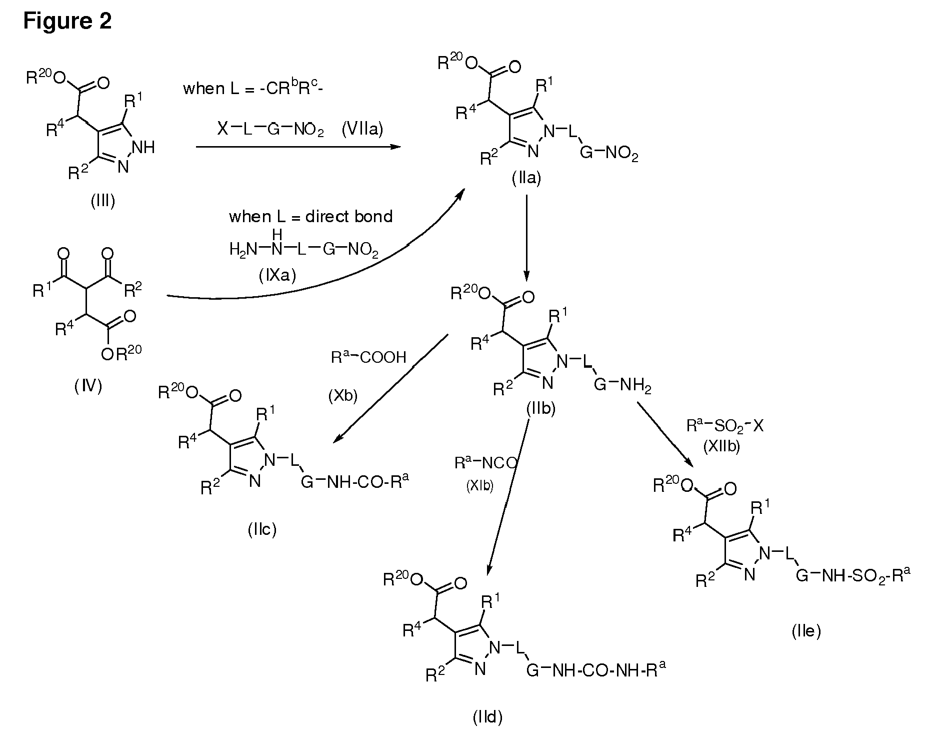

In the particular case where L represents a -CR^{b}R^{c}- group, compounds of formula (IIa) may be prepared by addition of an alkylating agent of formula (VIIa) wherein L represents a -CR^{b}R^{c}- group and X represents either a halogen atom such as chlorine, bromine or iodine or a leaving group such as methanesulphonate. The reaction is carried out by mixing the compound of formula (III) with an alkylating agent of formula (VIIa) wherein L and X are defined above, in a solvent such as dimethylformamide in the presence of a base such as potassium carbonate or sodium hydride at a temperature from ambient to 100ºC to yield the compound of formula (IIa). Alternatively, in the particular case where L represents a direct bond, compounds of general formula (IIa) may be prepared by the reaction of a diketone of formula (IV) with a hydrazine of formula (IXa) wherein L represents a direct bond. The reaction is carried out by mixing a compound of formula (IV) with a hydrazine of formula (IXa) in the presence or absence of a base such as triethylamine, in a solvent such as acetic acid at a temperature from ambient to the boiling point of the solvent to yield the compound of formula (IIa).

Compounds of general formula (IIb) may be prepared by a hydrogenation reaction of a compound of formula (IIa). The reaction is carried out by mixing the compound of formula (IIa) with a metal transition catalyst such as palladium in the presence of hydrogen in a solvent such as ethyl acetate at a temperature from ambient to the boiling point of the solvent to yield the compound of formula (IIb).

In the particular case where R³ represents a -NHCOR^{a}- group, compounds of formula (IIc) may be prepared using the known amide bond formation procedures. For example, the reaction is carried out by activation of a carboxylic acid of formula (Xb) wherein R^{a} is as defined above with a coupling agent such as N-ethyl-N'-(3-dimethylaminopropyl)carbodiimide hydrochloride in the presence of 1-hydroxybenzotriazole or O-(7-azabenzotriazol-1-yl)-N, N, N', N'-tetramethyluronium hexafluorophosphate in the presence of a base, such as diisopropylethylamine in a solvent such as N,N-dimethylformamide followed by addition of compounds of formula (IIb) at a temperature from ambient to the boiling point of the solvent to yield compound of formula (IIc).

In the particular case where R³ represents a -NHCONHR^{a} group, compounds of formula (IId) may be prepared using the known urea bond formation procedures. For example, the reaction is carried out by mixing the compound of formula (IIb) with an isocyanate of formula (Xlb) in a solvent such as dichloromethane at a temperature from ambient to the boiling point of the solvent to yield the compound of formula (IId).

In the particular case where R³ represents a -NHSO₂R^{a} group, compounds of formula (IIe) may be prepared by condensation of compound of formula (IIb) with a sulphonylating agent of formula (Xllb) wherein X represents a halogen atom such as chlorine. The reaction is carried out by mixing the compound of formula (IIb) with the sulphonylating agent of formula (Xllb) wherein X is defined above in the presence of a catalyst such as copper (I) oxide in a solvent such as acetonitrile at a temperature from ambient to the boiling point of the solvent to yield the compound of formula (IIe).

In the particular case where R³ represents a -CONHR^{a} group and n is 1, or in another particular case where R³ represents a -COR^{a} group and where R^{a} represents a 3- to 7-membered heterocyclyl group containing at least one heteroatom selected from N, S and O, compounds of general formula (II) may be prepared following the synthetic scheme depicted in Figure 3.

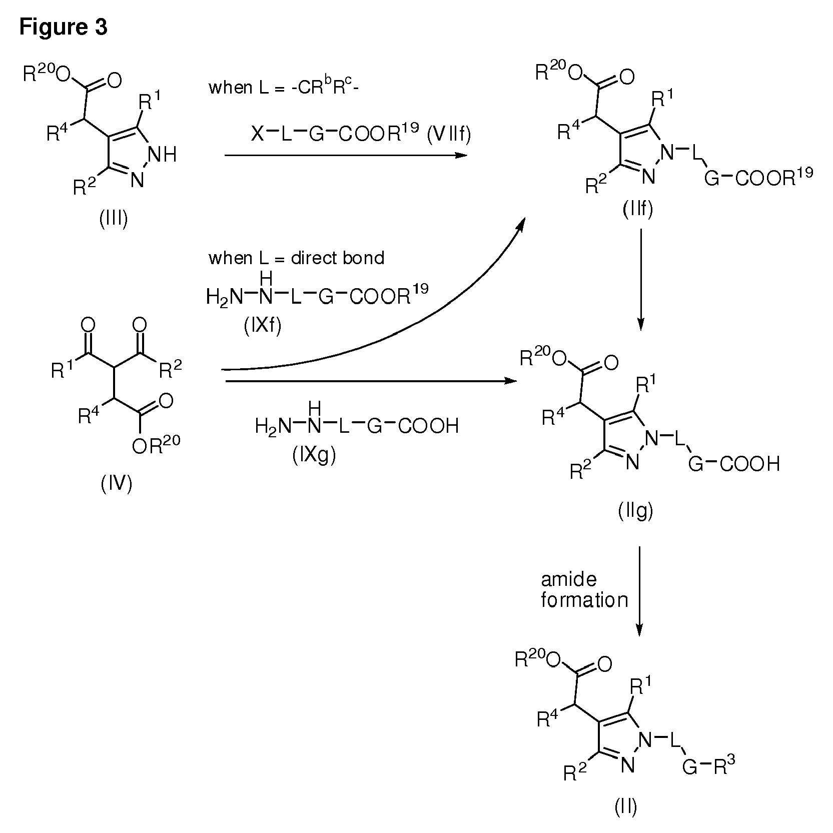

In the particular case where L represents a -CR^{b}R^{c}- group, compounds of formula (IIf) wherein R²⁰ is as defined above and R¹⁹ represent an alkyl group such as methyl, ethyl, propyl, benzyl, or tert-butyl may be prepared by addition of an alkylating agent of formula (Vllf) wherein L represents a -CR^{b}R^{c}- group and X represents either a halogen atom such as chlorine, bromine or iodine. The reaction is carried out by mixing the compound of formula (III) with an alkylating agent of formula (Vllf) wherein L and X are defined above, in a solvent such as dimethylformamide in the presence of a base such as potassium carbonate or sodium hydride to yield the compound of formula (IIf) at a temperature from ambient to 100 ºC to yield the compound of formula (IIf). Alternatively, in the particular case where L represents a direct bond, compounds of general formula (IIf) wherein R¹⁹ is as defined above may be prepared by the reaction of a diketone of formula (IV) with a hydrazine of formula (IXf) wherein L represents a direct bond. The reaction is carried out by mixing a compound of formula (IV) with a hydrazine of formula (IXf) in the presence or absence of a base such as triethylamine, in a solvent such as acetic acid at a temperature from ambient to the boiling point of the solvent to yield the compound of formula (IIf).

Compound of general formula (IIg) wherein R²⁰ and R¹⁹ separately represent an alkyl group such as methyl, ethyl, propyl, benzyl or tert-butyl may be prepared by hydrolysis of a single ester group of a compound of formula (IIf). The reaction is carried out by mixing a compound of formula (IIf) wherein R²⁰ and R¹⁹ are defined as above with a base such as lithium hydroxide in a solvent such as tetrahydrofuran or water or mixtures thereof at a temperature from 0ºC to the boiling point of the solvent to yield the compound of formula (IIg).

Alternatively, compounds of general formula (IIg) wherein R²⁰ is as defined above may be prepared by the reaction of a diketone of formula (IV) with a hydrazine of formula (IXg) wherein L represents a direct bond. The reaction is carried out by mixing a compound of formula (IV) with a hydrazine of formula (IXg) in the presence or absence of a base such as triethylamine, in a solvent such as acetic acid at a temperature from ambient to the boiling point of the solvent to yield the compound of formula (IIg).

Compounds of formula (II) may be prepared using the known amide bond formation procedures. The reaction is carried out by activation of the carboxylic acid of formula (IIg) with a coupling agent such as N-ethyl-N'-(3-dimethyl-aminopropyl)carbodiimide hydrochloride in the presence of 1-hydroxybenzotriazole or O-(7-azabenzotriazol-1-yl)-N,N,N',N'-tetramethyluronium hexafluorophosphate in the presence of a base, such as diisopropylethylamine in a solvent such as N,N-dimethylformamide followed by addition of an appropriate amine at a temperature from ambient to the boiling point of the solvent to yield compound of formula (II).

### PREPARATION EXAMPLES

The syntheses of the compounds of the invention are illustrated by the following Examples (1 to 31) including Preparation Examples (1 to 63) which do not limit the scope of the invention in any way.

### General

Reagents, starting materials, and solvents were purchased from commercial suppliers and used as received. Concentration or evaporation refer to evaporation under vacuum using a Büchi rotatory evaporator.

Reaction products were purified, when necessary, by flash chromatography on silica gel (40-63 µm) with the solvent system indicated. Purifications in reverse phase were made in a Biotage SP1® automated purification system equipped with a C18 column and using a gradient of water/acetonitrile/MeOH (1:1) (0.1% v/v ammonium formate both phases) from 0% to 100% acetonitrile/MeOH (1:1) in 40 column volumes. The appropriate fractions were collected and the solvents evaporated under reduced pressure and/or liofilized.

Gas chromatography was performed using a Thermo Trace Ultra gas chromatograph, coupled to a DSQ mass detector. Injections were performed on a split/splitless injector and a HP-1MS was the capillary column. Mass spectra were obtained by electron impact ionisation at 70eV.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector.

The chromatographic separations were obtained using a Waters 2795 system equipped with a Symmetry C18 (2.1 x 50 mm, 3.5 µM) column for methods A, B and C and a Symmetry C18 (2.1 x 100 mm, 3.5 µM) for method D. The mobile phases were (B): formic acid (0.4 mL), ammonia (0.1 mL), methanol (500 mL) and acetonitrile (500 mL) and (A): formic acid (0.5 mL), ammonia (0.125 mL) and water (1000 mL) (A), the gradients are specified in the following table for each method used.

| Method | Run time | 0% B | 0 to 95% B | 95% B |
|---|---|---|---|---|
| A | 5 min | 0.2 min | 3 min | 0.8 min |
| B | 9 min | 0.5 min | 6.5 min | 1 min |
| C | 15 min | 0 min | 10.5 min | 1.5 min |
| D | 30 min | 0 min | 20 min | 4 min |

The flow rate was 0.8 mL/min for method A and 0.4 mL/min for method B, C and D. The injection volume was 5 microliter. A Waters 2996 diode array was used as a UV detector. Chromatograms were processed at 210 nM or 254 nM. Mass spectra of the chromatograms were acquired using positive and negative electrospray ionization in a Micromass ZMD or in a Waters ZQ detectors coupled to the HPLC.

¹H Nuclear Magnetic Resonance Spectra were recorded on a Varian Gemini-2000 spectrometer operating at a frequency of 300MHz for the ¹H spectra or in a Varian Mercury plus operating at a frequency of 400MHz for the ¹H spectra. Samples were dissolved in the specified deuterated solvent. Tetramethylsilane was used as reference. The ¹H NMR spectrum for the following compounds was not recorded: Preparations 9, 10, 11, 39, 51, 55, 59, 62 and 63 and Example 4.

Mass Spectra (*m*/*z*) were recorded on a Micromass ZMD or in a Waters ZQ mass spectrometer using ESI ionization. The mass spectrum for the following compounds was not recorded: Preparation 29.

### PREPARATIONS

### PREPARATION 1

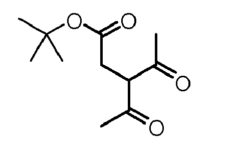

### tert-Butyl 3-acetyl-4-oxopentanoate

Sodium hydride (60% dispersion in oil, 1.5 g, 38 mmol) was first washed with hexanes to remove the oil and was then suspended in 20 ml tetrahydrofuran an cooled to 0 ºC in an ice-bath. A solution of 2,4-pentanedione (3.0 g, 30 mmol) dissolved in 20 ml tetrahydrofuran was added drop-wise and with stirring. Upon addition, the mixture was stirred for 1 h at 0 ºC. A solution of tert-butyl bromoacetate (5.0 g, 34 mmol) in 20 ml tetrahydrofuran was added drop-wise with stirring and the mixture was stirred overnight, warming to room temperature. The organics were concentrated under reduced pressure and the residue was quenched with ammonium chloride solution. The solution was extracted three times with ether and the combined organics were washed with brine, dried over sodium sulphate and evaporated under reduced pressure. The residue was purified using the SP1 Purification System (ethyl acetate-hexane gradient, 0:100 rising to 30:70) to give 4.0 g (19 mmol, 62%) of the title compound as a colourless oil. Purity 100%.
¹H NMR (400 MHz, CHLOROFORM-*d*) Ketone tautomer: δ ppm 4.05 (1 H, t, *J*=7.2 Hz), 2.78 (2 H, d, *J*=7.4 Hz), 2.13 (6 H, s), 1.40 (9 H, s). Enol tautomer: δ ppm 3.13 (2 H, s), 2.23 (6 H, s), 1.43 (9 H, s).
HPLC/MS (9 min) retention time 5.23 min.
LRMS: *m*/*z* 215 (M+1).

### PREPARATION 2

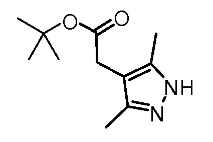

### tert-Butyl(3,5-dimethyl-1H-pyrazol-4-yl)acetate

The title compound from Preparation 1 (0.60 g, 2.8 mmol) was dissolved in 13 ml acetic acid. Hydrazine monohydrate (0.21 ml, 4.2 mmol) was added and the mixture was stirred for 1 h at room temperature. The mixture was concentrated under reduced pressure and the residue was partitioned between ether and water. The organic layer was washed with water, then with brine, and was dried over sodium sulphate. Evaporation under reduced pressure gave 0.58 g (2.7 mmol, 98%) of the title compound as a colourless oil. Purity 100%.
¹H NMR (400 MHz, CHLOROFORM-d) δ ppm 5.75 (1 H, br. s.), 3.25 (2 H, s), 2.23 (6 H, s), 1.42 (9 H, s).
HPLC/MS (9 min) retention time 5.03 min.
LRMS: *m*/*z* 211 (M+1).

### PREPARATION 3

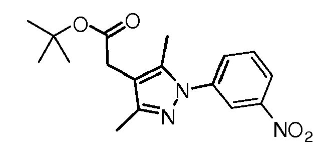

### tert-Butyl [3,5-dimethyl-1-(3-nitrophenyl)-1H-pyrazol-4-yl]acetate

The title compound of Preparation 1 (1.0 g, 4.7 mmol) and (3-nitrophenyl)hydrazine hydrochloride (1.4 g, 7.4 mmol) were suspended in 10 ml acetic acid. Triethylamine (1.0 ml, 7.0 mmol) was added and the mixture warmed to dissolve the solids. The mixture was stirred overnight at room temperature. The mixture was concentrated under reduced pressure and the residue was partitioned between ether and water. The organic layer was washed with water, then with brine, and was dried over sodium sulphate. Evaporation under reduced pressure gave an oil. The oil was washed twice with hot hexane, decanting each time. The remaining oil was evaporated under reduced pressure to give 1.4 g (4.2 mmol, 89%) of the title compound as a colourless oil. Purity 99%.
¹H NMR (400 MHz, CHLOROFORM-*d*) δ ppm 8.34 (1 H, t, *J*=2.1 Hz), 8.19 (1 H, dd, *J*=8.2, 2.0 Hz), 7.84 (1 H, dd, *J*=8.2, 1.5 Hz), 7.64 (1 H, t, *J*=8.2 Hz), 3.35 (2 H, s), 2.36 (3 H, s), 2.29 (3 H, s), 1.47 (9 H, s).
HPLC/MS (9 min) retention time 6.67 min.
LRMS: *m*/*z* 332 (M+1).

### PREPARATION 4

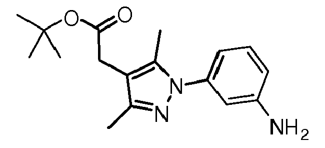

### tert-Butyl[1-(3-aminophenyl)-3,5-dimethyl-1H-pyrazol-4-yl]acetate

The title compound of Preparation 3 (1.0 g, 3.0 mmol) was dissolved in 10 ml ethyl acetate. 10% Palladium on carbon (0.1 g) was added and the mixture was stirred overnight under 25 p.s.i. atmosphere of hydrogen. The mixture was filtered through Celite® and the celite was washed with ethyl acetate. The combined organics were evaporated under reduced pressure to give 0.89 g (2.95 mmol, 99%) of the title compound as a colourless oil. Purity 100%.
¹H NMR (400 MHz, CHLOROFORM-*d*) δ ppm 7.21 (1 H, t, *J*=7.8 Hz), 6.96 (1 H, t, *J*=2.0 Hz), 6.85 (1 H, dd, *J*=7.8, 1.5 Hz), 6.81 (1 H, dd, *J*=8.2, 2.0 Hz), 3.31 (2 H, s), 2.28 (3 H, s), 2.21 (3 H, s), 1.45 (9 H, s).
HPLC/MS (9 min) retention time 5.87 min.
LRMS: *m*/*z* 302 (M+1).

### PREPARATION 5

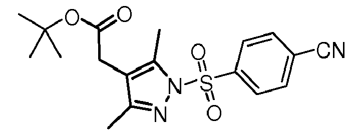

### tert-Butyl{1-[(4-cyanophenyl)sulfonyl]-3,5-dimethyl-1H-pyrazol-4-yl}acetate

Sodium hydride (60% dispersion in oil, 20 mg, 0.5 mmol) was then suspended in 5 ml tetrahydrofuran. A solution of the title compound of Preparation 2 (100 mg, 0.48 mmol) dissolved in 5 ml tetrahydrofuran was added and the mixture was stirred for 30 min at room temperature. 4-Cyanobenzene sulfonyl chloride (96 mg, 0.48 mmol) was added and the mixture was stirred overnight at room temperature. The mixture was concentrated under reduced pressure. The residue was purified using the SP1 Purification System (ethyl acetate-hexane) to give 70 mg (0.18 mmol, 38%) of the title compound as a colourless oil. Purity 98%.
¹H NMR (400 MHz, CHLOROFORM-*d*) δ ppm 8.05 (2 H, d, *J*=8.6 Hz), 7.81 (2 H, d, *J*=8.6 Hz), 3.21 (2 H, s), 2.47 (3 H, s), 2.19 (3 H, s), 1.38 (9 H, s).
HPLC/MS (9 min) retention time 6.42 min.
LRMS: *m*/*z* 376 (M+1).

### PREPARATION 6

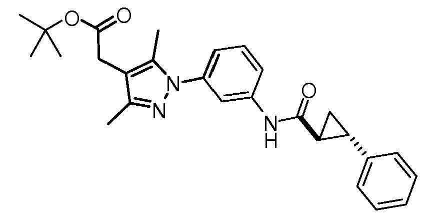

### tert-Butyl {3,5-dimethyl-1-[3-({[trans-2-phenylcyclopropyl]carbonyl}amino)-phenyl]-1H-pyrazol-4-yl}acetate

The title compound of Preparation 4 (200 mg, 0.66 mmol), *trans* 2-phenylcyclopropanecarboxylic acid (120 mg, 0.73 mmol), 1-hydroxybenzotriazole (120 mg, 0.87 mmol) and 1-(3-dimethylaminopropyl)-3-ethylcarbodiimide hydrochloride (165 mg, 0.86 mmol) were dissolved in 10 ml dimethylformamide and the mixture was stirred for 3 days at room temperature. The mixture was concentrated under reduced pressure and purified directly by reverse-phase chromatography using the SP1 Purification System to give 200 mg (0.45 mmol, 68%) of the title compound as a colourless oil. Purity 100%.
¹H NMR (400 MHz, CHLOROFORM-*d*) δ ppm 8.79 (1 H, br. s.), 7.67 (1 H, d, *J*=7.8 Hz), 7.56 (1 H, br. s.), 7.17 - 7.31 (4 H, m), 7.00 (2 H, d, *J*=7.4 Hz), 6.79 (1 H, d, *J*=7.4 Hz), 3.30 (2 H, s), 2.39 - 2.52 (1 H, m), 2.21 (3 H, s), 2.21 (3 H, s), 1.58 - 1.72 (2 H, m), 1.46 (9 H, s), 1.16 - 1.30 (1 H, m).
HPLC/MS (30 min) retention time 16.92 min.
LRMS: *m*/*z* 446 (M+1).

### PREPARATION 7

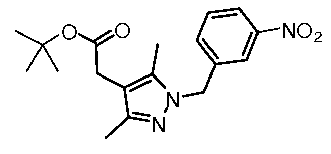

### tert-Butyl[3,5-dimethyl-1-(3-nitrobenzyl)-1H-pyrazol-4-yl]acetate

The title compound of Preparation 2 (1.0 g, 4.8 mmol) was dissolved in 20 ml dimethylformamide. Potassium carbonate (2.6 g, 4.0 mmol) and 3-nitrobenzyl bromide (2.0 g 2.0 mmol) were added and the mixture was stirred overnight at room temperature. A further 2.6 g potassium carbonate and 2.0 g 3-nitrobenzyl bromide were added and the mixture was stirred overnight at room temperature. The mixture was concentrated under reduced pressure and the residue was partitioned between water and dichloromethane. The organic layer was washed with brine and was dried over sodium sulphate. Evaporation under reduced pressure gave an oil which was purified by reverse-phase chromatography using the SP1 Purification System to give 1.3 g (3.7 mmol, 78%) of the title compound as an oil. Purity 99%.
¹H NMR (400 MHz, CHLOROFORM-*d*) δ ppm 8.12 (1 H, d, *J*=8.2 Hz), 7.93 (1 H, s), 7.49 (1 H, t, *J*=7.8 Hz), 7.35 (1 H, d, *J*=7.8 Hz), 5.32 (2 H, s), 3.28 (2 H, s), 2.24 (3 H, s), 2.15 (3 H, s), 1.43 (9 H, s).
HPLC/MS (9 min) retention time 6.55 min.
LRMS: *m*/*z* 346 (M+1).

### PREPARATION 8

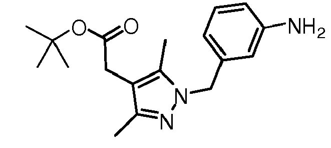

### tert-Butyl [1-(3-aminobenzyl)-3,5-dimethyl-1H-pyrazol-4-yl]acetate

The title compound of Preparation 7 (0.85 g, 2.5 mmol) was treated according to the method of Preparation 4 to give 0.62 g (1.9 mmol, 78%) of the title compound as a colourless oil. Purity 98%.
¹H NMR (400 MHz, CHLOROFORM-*d*) δ ppm 7.05 (1 H, t, *J*=7.6 Hz), 6.54 (1 H, dd, *J*=8.0, 1.8 Hz), 6.44 (1 H, d, *J*=7.8 Hz), 6.33 (1 H, s), 5.12 (2 H, s), 3.62 (2 H, br. s.), 3.25 (2 H, s), 2.23 (3 H, s), 2.10 (3 H, s), 1.42 (9 H, s). HPLC/MS (9 min) retention time 5.47 min.
LRMS: *m*/*z* 316 (M+1).

### PREPARATION 9

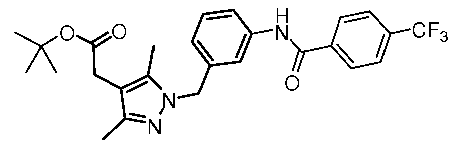

### tert-Butyl [3,5-dimethyl-1-(3-{[4-(trifluoromethyl)benzoyl]amino}benzyl)-1H-pyrazol-4-yl]acetate

The title compound of Preparation 8 (50 mg, 0.16 mmol) was dissolved in 2 ml dichloromethane. Polystyrene-supported morpholine (100 mg, Aldrich, Loading 2.5-4 mmol/g) was added and the mixture agitated for 5 min. 4-Trifluoromethylbenzoyl chloride (0.03 ml, 0.20 mmol) was added and the mixture was agitated overnight at room temperature. Polystyrene-supported trisamine (50 mg, Argonaut, Loading 4.23 mmol/g) was added and the mixture was agitated for 2 h. The mixture was filtered and the filtrate evaporated under reduced pressure. The residue was purified by reverse-phase chromatography using the SP1 Purification System to give 55 mg (0.112 mmol, 70%) of the title compound as an oil. Purity 100%.
HPLC/MS (9 min) retention time 7.20 min.
LRMS: *m*/*z* 488 (M+1).

### PREPARATION 10

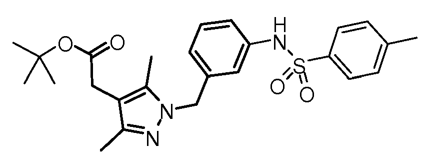

### tert-Butyl [3,5-dimethyl-1-(3-{[(4-methylphenyl)sulfonyl]amino}benzyl)-1H-pyrazol-4-yl]acetate

The title compound of Preparation 8 (50 mg, 0.16 mmol) and tosyl chloride (36 mg, 0.19 mmol) was reacted according to the method of Preparation 9 to give, after purification by reverse-phase chromatography using the SP1 Purification System, 20 mg (0.042 mmol, 27%) of the title compound as an oil. Purity 100%.
HPLC/MS (9 min) retention time 6.77 min.
LRMS: *m*/*z* 470 (M+1).

### PREPARATION 11

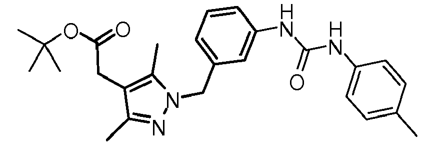

### tert-Butyl {3,5-dimethyl-1-[3-({[(4-methylphenyl)amino]carbonyl}amino)benzyl]-1H-pyrazol-4-yl}acetate

The title compound of Preparation 8 (50 mg, 0.16 mmol) and 4-methylbenzene isocyanate (0.025 ml, 0.19 mmol) were dissolved in 2 ml dichloromethane and the mixture was agitated overnight at room temperature. Polystyrene-supported trisamine (50 mg, Argonaut, Loading 4.23 mmol/g) was added and the mixture was agitated for 2 h. The mixture was filtered and the filtrated evaporated under reduced pressure to give 155 mg of the crude title compound as an oil. Purity 66%.
HPLC/MS (15 min) retention time 9.18 min.
LRMS: *m*/*z* 449 (M+1).

### PREPARATION 12

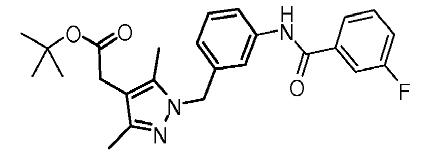

### tert-Butyl (1-{3-[(3-fluorobenzoyl)amino]benzyl}-3,5-dimethyl-1H-pyrazol-4-yl)acetate

The title compound of Preparation 8 (66 mg, 0.21 mmol), 3-fluorobenzoic acid (32 mg, 0.23 mmol), 1-hydroxybenzotriazole (37 mg, 0.27 mmol) and 1-(3-dimethylaminopropyl)-3-ethylcarbodiimide hydrochloride (52 mg, 0.27 mmol) were reacted according to the method of Preparation 6 to give 70 mg (0.16 mmol, 76%) of the title compound as a solid. Purity 100%.
¹H NMR (400 MHz, CHLOROFORM-*d*) δ ppm 7.90 (1 H, br. s.), 7.69 (1 H, d, *J*=7.4 Hz), 7.53 - 7.66 (2 H, m), 7.46 (1 H, d, *J*=5.9 Hz), 7.20 - 7.35 (2 H, m), 7.15 (1 H, br. s.), 6.85 (1 H, d, *J*=6.6 Hz), 5.23 (2 H, s), 3.27 (2 H, br. s.), 2.22 (3H, s), 2.13 (3H, s), 1.44 (9 H, s).
HPLC/MS (9 min) retention time 6.80 min.
LRMS: *m*/*z* 438 (M+1).

### PREPARATION 13

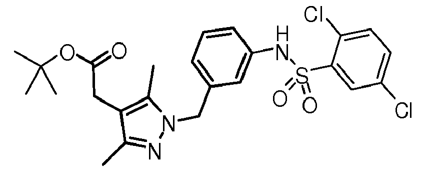

### tert-Butyl [1-(3-{[(2,5-dichlorophenyl)sulfonyl]amino}benzyl)-3,5-dimethyl-1H-pyrazol-4-yl]acetate

The title compound of Preparation 8 (66 mg, 0.21 mmol) and 2,5-dichlorobenzene-1-sulfonyl chloride (51 mg, 0.21 mmol) were dissolved in 10 ml acetonitrile. Copper(II) oxide (1 mg) was added and the mixture was stirred overnight. The mixture was concentrated under reduced pressure. The residue was partitioned between water and dichloromethane. The organic layer was washed with sodium bicarbonate solution and was evaporated to dryness. The residue was purified by reverse-phase chromatography using the SP1 purification system to give 40 mg (0.074 mmol, 36%) of the title compound. Purity 98%.
¹H NMR (400 MHz, CHLOROFORM-*d*) δ ppm 7.95 (1 H, s), 7.41 (2 H, s), 7.13 - 7.21 (2 H, m), 7.03 (1 H, d, *J*=9.0 Hz), 6.77 (1 H, d, *J*=7.8 Hz), 6.72 (1 H, s), 5.13 (2 H, s), 3.26 (2 H, s), 2.21 (3 H, s), 2.03 (3 H, s), 1.43 (9 H, s).
HPLC/MS (9 min) retention time 7.03 min.
LRMS: *m*/*z* 524 (M+1).

### PREPARATION 14

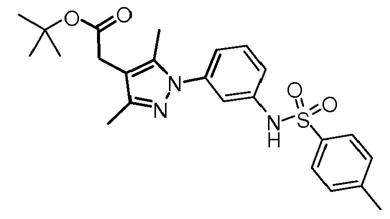

### tert-Butyl [3,5-dimethyl-1-(3-{[(4-methylphenyl)sulfonyl]amino}phenyl)-1H-pyrazol-4-yl]acetate

The title compound of Preparation 4 (105 mg, 0.35 mmol), tosyl chloride (67 mg, 0.35 mmol) and copper(II) oxide (2.8 mg, 0.04 mmol) were reacted together according to the method of Preparation 13 to give 90 mg (0.20 mmol, 57%) of the title compound as a solid. Purity 100%.
¹H NMR (400 MHz, CHLOROFORM-*d*) δ ppm 7.65 (2 H, d, *J*=8.2 Hz), 7.31 (1 H, t, *J*=8.0 Hz), 7.22 (2 H, d, *J*=8.6 Hz), 7.17 - 7.20 (1 H, m), 7.10 (2 H, br. s.), 6.74 (1 H, br. s.), 3.31 (2 H, s), 2.38 (3 H, s), 2.25 (3 H, s), 2.20 (3 H, s), 1.46 (9 H, s).
HPLC/MS (9 min) retention time 6.88 min.
LRMS: *m*/*z* 456 (M+1).

### PREPARATION 15

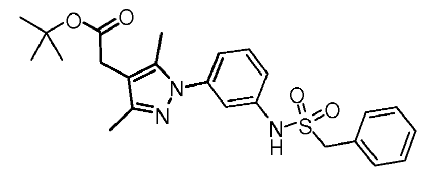

### tert-Butyl (1-{3-[(benzylsulfonyl)amino]phenyl}-3,5-dimethyl-1H-pyrazol-4-yl)acetate

The title compound of Preparation 4 (105 mg, 0.35 mmol), phenylmethylsulfonyl chloride (66 mg, 0.35 mmol) and copper(II) oxide (2.8 mg, 0.04 mmol) were reacted together according to the method of Preparation 13 to give 100 mg (0.175 mmol, 51 %) of the title compound. Used as such without further purification. Purity 80%.
¹H NMR (400 MHz, CHLOROFORM-*d*) δ ppm 7.32 - 7.44 (4 H, m), 7.22 - 7.32 (4 H, m), 7.05 (1 H, d, *J*=8.6 Hz), 6.38 (1 H, s), 4.38 (2 H, s), 3.33 (2 H, s), 2.30 (3 H, s), 2.28 (3 H, s), 1.47 (9 H, s).
HPLC/MS (9 min) retention time 6.78 min.
LRMS: *m*/*z* 456 (M+1).

### PREPARATION 16

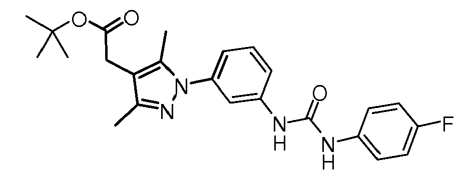

### tert-Butyl {1-[3-({[(4-fluorophenyl)amino]carbonyl}amino)phenyl]-3,5-dimethyl-1H-pyrazol-4-yl}acetate

The title compound of Preparation 4 (115 mg, 0.38 mmol) and 4-fluorophenyl isocyante (50 mg, 0.44 mmol) were dissolved in 5 ml dichloromethane and the mixture was stirred overnight. The mixture was concentrated under reduced pressure to give 140 mg (0.32 mmol, 84%) of the title compound as a solid. Used as such without further purification. Purity 95%.
¹H NMR (400 MHz, CHLOROFORM-d) δ ppm 8.15 (1 H, s), 7.96 (1 H, s), 7.24 - 7.34 (2 H, m), 7.07 - 7.22 (3 H, m), 6.80 - 6.93 (3 H, m), 3.36 (2 H, s), 2.28 (3 H, s), 2.21 (3 H, s), 1.48 (9 H, s).
HPLC/MS (9 min) retention time 6.95 min.
LRMS: m/z 439 (M+1).

### PREPARATION 17

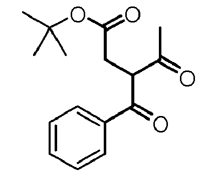

### tert-Butyl 3-benzoyl-4-oxopentanoate

1-Phenylbutane-1,3-dione (2.0 g, 12.4 mmol) was treated with sodium hydride (60% dispersion in oil, 0.73 g, 18.5 mmol) and tert-butyl bromoacetate (2.2 g, 14.7 mmol) according to the method of Preparation 1 without purification to give 3.29 g (9.44 mmol, 76%) of the crude title compound. Used as such without further purification. Purity 79%.
¹H NMR (400 MHz, CHLOROFORM-d) δ ppm 8.02 (2 H, d, J=7.4 Hz), 7.61 (1 H, t, J=7.2 Hz), 7.51 (2 H, t, J=7.6 Hz), 4.94 (1 H, t, J=7.0 Hz), 2.95 (1 H, dd, J=16.5, 7.0 Hz), 2.89 (0 H, dd, J=16.5, 7.0 Hz), 2.17 (3 H, s), 1.40 (9 H, s). HPLC/MS (9 min) retention time 6.14 min.
LRMS: m/z 221 (M+1).

### PREPARATION 18

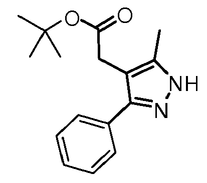

### tert-Butyl (5-methyl-3-phenyl-1H-pyrazol-4-yl)acetate

The crude title compound of Preparation 17 (3.3 g) was treated with hydrazine hydrate (0.64 ml, 12.8 mmol) according to the method of Preparation 2 followed by purification by reverse-phase chromatography using the SP1 Purification system to give 1.94 g (7.1 mmol, 57% yield over two steps) of the title compound as a white solid. Purity 100%.
¹H NMR (400 MHz, CHLOROFORM-d) δ ppm 7.58 (2 H, d, J=7.0 Hz), 7.31 - 7.45 (2 H, m), 3.41 (2 H, s), 2.16 - 2.32 (3 H, m), 1.41 (9 H, s)
HPLC/MS (15 min) retention time 7.67 min.
LRMS: *m*/*z* 217 (M+1).

### PREPARATION 19

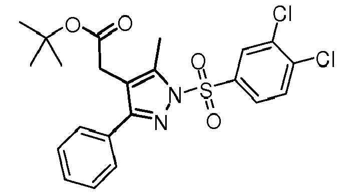

### tert-Butyl {1-[(3,4-dichlorophenyl)sulfonyl]-5-methyl-3-phenyl-1H-pyrazol-4-yl}acetate

The title compound of Preparation 18 (160 mg, 0.59 mmol) was reacted with sodium hydride (60% dispersion in oil, 28 mg, 0.70 mmol) and 3,4-dichlorobenzene sulfonyl chloride (92 mg, 0.59 mmol) according to the method of Preparation 5 to give 165 mg (0.34 mmol, 58%) of the title compound as a solid. Purity 100%.
¹H NMR (400 MHz, CHLOROFORM-d) δ ppm 8.11 (1 H, d, J=2.0 Hz), 7.85 (1 H, dd, J=8.6, 2.0 Hz), 7.61 (1 H, d, J=8.6 Hz), 7.54 - 7.59 (2 H, m), 7.37 - 7.46 (3 H, m), 3.38 (2 H, s), 2.56 (3 H, s), 1.35 (9 H, s).
HPLC/MS (9 min) retention time 7.62 min.
LRMS: *m*/*z* 481 (M+1).

### PREPARATION 20

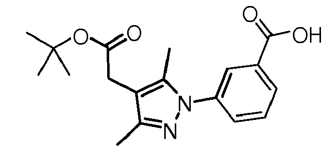

### 3-[4-(2-tert-Butoxy-2-oxoethyl)-3,5-dimethyl-1H-pyrazol-1-yl]benzoic acid

The title compound of Preparation 1 (1.5 g, 6.9 mmol) and 3-hydrazinylbenzoic acid (1.0 g, 6.6 mmol) were suspended in 125 ml acetic acid. The mixture was stirred overnight at room temperature. The mixture was concentrated under reduced pressure and the residue was purified by reverse-phase chromatography using the SP1 Purification to give 1.0 g (3.0 mmol, 46%) of the title compound as a solid. Purity 100%.
¹H NMR (400 MHz, CHLOROFORM-*d*) δ ppm 8.95 - 9.38 (1 H, m), 8.16 (1 H, s), 8.06 (1 H, d, *J*=7.8 Hz), 7.68 (1 H, d, *J*=7.8 Hz), 7.54 (1 H, t, *J*=7.8 Hz), 3.35 (2 H, s), 2.32 (3 H, s), 2.30 (3 H, s), 1.47 (9 H, s).
HPLC/MS (9 min) retention time 6.13 min.
LRMS: *m*/*z* 331 (M+1).

### PREPARATION 21

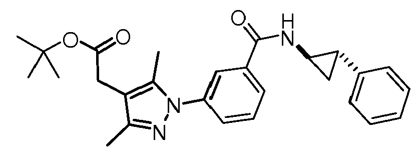

### tert-Butyl {3,5-dimethyl-1-[3-({[trans-2-phenylcyclopropyl]amino}carbonyl)-phenyl]-1H-pyrazol-4-yl}acetate

The title compound of Preparation 20 (100 mg, 0.30 mmol), trans 2-phenylcyclopropylamine (80 mg, 0.60 mmol), 1-hydroxybenzotriazole (60 mg, 0.44 mmol) and 1-(3-dimethylaminopropyl)-3-ethylcarbodiimide hydrochloride (90 mg, 0.47 mmol) were reacted according to the method of Preparation 6 to give 60 mg (0.13 mmol, 44%) of the title compound as a solid. Purity 100%.
¹H NMR (400 MHz, CHLOROFORM-d) δ ppm 7.74 - 7.83 (2 H, m), 7.44 - 7.55 (2 H, m), 7.28 (2 H, t, J=7.0 Hz), 7.19 (3 H, d, J=7.0 Hz), 6.62 (1 H, br. s.), 3.32 (2 H, s), 3.01 - 3.11 (1 H, m), 2.26 (6 H, s), 2.16 (1 H, ddd, J=9.3, 6.2, 3.3 Hz), 1.45 (9 H, s), 1.34 (1 H, q, J=6.5 Hz), 1.22 - 1.30 (1 H, m).
HPLC/MS (9 min) retention time 6.93 min.
LRMS: *m*/*z* 446 (M+1).

### PREPARATION 22

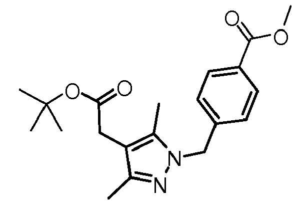

### Methyl 4-{[4-(2-tert-butoxy-2-oxoethyl)-3,5-dimethyl-1H-pyrazol-1-yl]methyl}benzoate

The title compound of Preparation 2 (500mg, 2.3 mmol) was dissolved in 15 ml dimethylformamide. Sodium hydride (60% dispersion in oil, 124 mg, 3.1 mmol) previously washed with pentane was added in portions and the mixture was stirred 30 minutes at room temperature. Methyl 4-(bromomethyl)benzoate dissolved in dimethylformamide was then added and the mixture was agitated for 2h. The mixture is poured into water and the solution was extracted three times with ether. The combined organics were washed with brine, dried over sodium sulphate and evaporated under reduced pressure. The residue was purified using the SP1 Purification System (ethyl acetate-hexane gradient, 0:100 rising to 70:30) to give 0.62 g (1.72 mmol, 73%) of the title compound as a yellow oil. Purity 99%.
¹H NMR (400 MHz, CHLOROFORM-d) δ ppm 1.41 (s, 9 H), 2.09 (s, 3 H), 2.23 (s, 3 H), 3.25 (s, 2 H), 3.89 (s, 3 H), 5.27 (s, 2 H), 7.07 (d, J=8.21 Hz, 2 H), 7.96 (d, J=8.21 Hz, 2 H).
HPLC/MS (9 min) retention time 6.50 min.
LRMS: *m*/*z* 359(M+1).

### PREPARATION 23

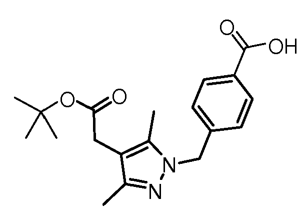

### 4-{[4-(2-tert-Butoxy-2-oxoethyl)-3,5-dimethyl-1H-pyrazol-1-yl]methyl}benzoic acid

The title compound of Preparation 22 (0.62 g, 1.7 mmol) was dissolved in 15 ml tetrahydrofuran and 11 ml of water. Lithium hydroxide (0.36 mg, 8.6 mmol) was added and the mixture was agitated at room temperature for 2 h. The mixture was concentrated under reduced pressure and the residue was acidified with acetic acid to pH 5. The solution was extracted three times with ethyl acetate. The combined organics were washed with brine, dried over sodium sulphate and evaporated under reduced pressure to give 360 mg (1.04 mmol, 57%) of the title compound as a white solid. Used as such without further purification. Purity 94%.
¹H NMR (400 MHz, CHLOROFORM-d) δ ppm 1.42 (s, 9 H), 2.11 (s, 3 H), 2.25 (s, 3 H), 3.27 (s, 2 H), 5.32 (s, 2 H), 7.09 (d, J=8.21 Hz, 2 H), 7.99 (d, J=8.21 Hz, 2 H).
HPLC/MS (9 min) retention time 5.98 min.
LRMS: *m*/*z* 345 (M+1).

### PREPARATION 24

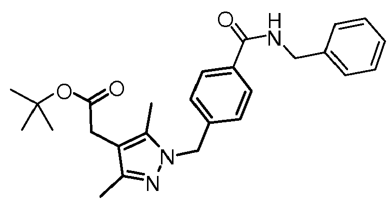

### tert-Butyl (1-{4-[(benzylamino)carbonyl]benzyl}-3,5-dimethyl-1H-pyrazol-4-yl)acetate

The title compound of Preparation 23 (80 mg, 0.22 mmol), diisopropylethylamine (84 µl, 0.48 mmols) and O-(7-azabenzotriazol-1-yl)-N,N,N',N'-tetramethyluronium hexafluorophosphate (HATU) (83 mg, 0.22 mmol) were dissolved in 3 ml dimethylformamide and the mixture was agitated for 20 min. Benzylamine (28 mg, 0.26 mmol) was added and the mixture was agitated for 2 h. The mixture was concentrated under reduced pressure and the residue was partitioned between water and sodium bicarbonate solution. The organic layer was washed with water, then with brine, and was dried over sodium sulphate. Evaporation under reduced pressure gave an oil. The residue was purified using the SP1 Purification System (ethyl acetate-hexane gradient, 0:100 rising to 60:40) to give 49 mg (0.11 mmol, 52%) of the title compound as an oil. Purity 100%.
¹H NMR (400 MHz, CHLOROFORM-d) δ ppm 1.42 (s, 9 H), 2.09 (s, 3 H), 2.22 (s, 3 H), 3.25 (s, 2 H), 4.63 (d, J=5.86 Hz, 2 H), 5.25 (s, 2 H), 6.38 (br. s., 1 H), 7.07 (d, J=8.21 Hz, 2 H), 7.27 - 7.40 (m, 5 H), 7.71 (d, J=8.21 Hz, 2 H).
HPLC/MS (9 min) retention time 6.48 min.
LRMS: *m*/*z* 434 (M+1).

### PREPARATION 25

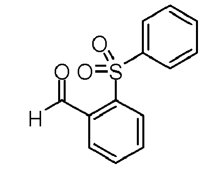

### 2-(Phenylsulfonyl)benzaldehyde

2-fluorobenzaldehyde (2.36 g, 18.4 mmol) and sodium benzenesulfinate (3.4 g, 20 mmol) were suspended in 20 ml dimethylsufoxide. The mixture was heated at 100ºC for 4 days. The mixture was concentrated under reduced pressure and the residue was partitioned between water and ethyl acetate. The aqueous layer was extracted three times with ethyl acetate. The combined organics were washed with water and brine and dried over magnesium sulphate and evaporated. The residue was purified using the SP1 Purification System (ethyl acetate-hexane gradient, 0:100 rising to 30:70) to give 1.98 g (8.0 mmol, 47%) of the title compound as a white solid. Purity 100%.
¹H NMR (400 MHz, DMSO-d6) δ ppm 7.67 (t, J=7.62 Hz, 2 H), 7.75 (t, 1 H), 7.88 - 7.99 (m, 3 H), 8.02 (d, J=7.42 Hz, 2 H), 8.20 (d, J=7.82 Hz, 1 H), 10.68 (s, 1H).
HPLC/MS (9 min) retention time 5.47 min.
LRMS: *m*/*z* 247 (M+1).

### PREPARATION 26

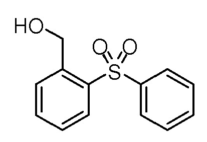

### [2-(Phenylsulfonyl)phenyl]methanol

The title compound of Preparation 25 (1.98 g, 8.0 mmol) was suspended in 16 ml methanol. Sodium borohydride (150 mg, 3.86 mmol) was added in portions and the mixture was stirred at room temperature for 30 min. 5% Hydrochloric acid solution (4 ml) was added and the mixture was evaporated. The residue was taken up in water and the aqueous was extracted two times with dichloromethane. The combined organics were washed with brine and dried over sodium sulphate. Filtration and evaporation gave 1.9 g (7.3 mmol, 95%) of the title compound as a solid. Used as such without further purification. Purity 96%.
¹H NMR (400 MHz, CHLOROFORM-d) δ ppm 7.27 (s, 2 H), 7.49 - 7.58 (m, 4 H), 7.62 (q, J=7.42 Hz, 2 H), 7.90 (d, J=7.42 Hz, 2 H), 8.15 (d, J=7.82 Hz, 1 H).
HPLC/MS (9 min) retention time 4.97 min.
LRMS: *m*/*z* 249 (M+1).

### PREPARATION 27

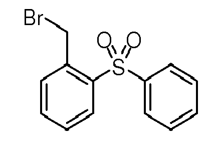

### 1-(Bromomethyl)-2-(phenylsulfonyl)benzene.

The title compound of Preparation 26 (1.9 g, 7.3 mmol) was suspended in 20 ml chlorobenzene. Thionyl bromide (1.46 ml, 18.3 mmol) was slowly added and the mixture was heated at 110ºC for 1 h. The mixture was concentrated under reduced pressure and the residue was partitioned between water and dichloromethane. The aqueous phase was basified with potassium carbonate and extracted three times with dichloromethane. The combined organics were washed with water, brine and dried over sodium sulphate and evaporated. The residue was purified by column chromatography (ethyl acetate-hexane gradient, 0:100 rising to 8:92) to give 1.95 g (5.89 mmol, 80%) of the title compound as an orange oil. Purity 94%.
¹H NMR (400 MHz, CHLOROFORM-d) δ ppm 7.26 (s, 2 H), 7.47 - 7.64 (m, 6 H), 7.92 (d, J=7.42 Hz, 2 H), 8.19 (d, J=8.21 Hz, 1 H).
HPLC/MS (9 min) retention time 6.03 min.
LRMS: m/z 330 (M+1).

### PREPARATION 28

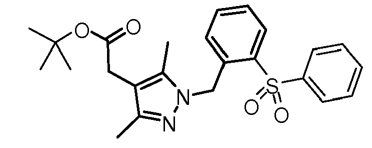

### tert-Butyl {3,5-dimethyl-1-[2-(phenylsulfonyl)benzyl]-1H-pyrazol-4-yl}acetate

The title compound of Preparation 2 (90 mg, 0.43 mmol) was reacted with sodium hydride (60% dispersion in oil, 22 mg, 0.55 mmol) and the title compound of Preparation 27 (172 mg, 0.52 mmol) according to the method of Preparation 22. The residue was purified using the SP1 Purification System (ethyl acetate-hexane gradient, 0:100 rising to 15:85) to give 125 mg (0.28 mmol, 66%) of the title compound as an oil. Purity 99 %.
¹H NMR (400 MHz, CHLOROFORM-d) δ ppm 1.41 (s, 9 H), 1.87 (s, 3 H), 2.20 (s, 3 H), 3.25 (s, 2 H), 5.48 (s, 2 H), 6.32 - 6.40 (m, 1 H), 7.42 - 7.48 (m, 2 H), 7.53 (t, J=7.42 Hz, 2 H), 7.60 (t, 1 H), 7.90 (d, J=7.42 Hz, 2 H), 8.21 - 8.26 (m, 1 H).
HPLC/MS (9 min) retention time 6.85 min.
LRMS: *m*/*z* 441 (M+1).

### PREPARATION 29

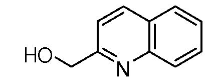

### Quinolin-2-ylmethanol

Quinoline-2-carboxaldehyde (6.36 g, 40.7 mmol) was treated with sodium borohydride (770 mg, 20.2 mmol) according to the method of Preparation 26 to give 6.05 g (38 mmol, 94%) of the title compound as a yellow oil. Purity 100%.
¹H NMR (400 MHz, CHLOROFORM-d) δ ppm 4.53 (br. s., 1 H), 4.93 (s, 2 H), 7.30 (d, J=8.60 Hz, 1 H), 7.55 (t, J=7.23 Hz, 1 H), 7.74 (t, J=7.03 Hz, 1 H), 7.84 (d, J=7.82 Hz, 1 H), 8.08 (d, J=8.21 Hz, 1 H), 8.15 (d, J=8.60 Hz, 1 H).

### PREPARATION 30

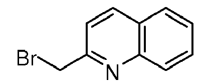

### 2-(Bromomethyl)quinoline

The title compound of Preparation 29 (3.68 g, 23.1 mmol) was suspended in 60 ml toluene. Thionyl bromide (12.0 g, 57.8 mmol) was slowly added and the mixture was heated at 110ºC for 1 h. The mixture was concentrated under reduced pressure and the residue was partitioned between water and dichloromethane. The aqueous phase was basified with potassium carbonate and extracted three times with dichloromethane. The combined organics were washed with water, brine and dried over sodium sulphate and evaporated. The residue was purified by column chromatography (ethyl acetate-hexane, 5:95) to give 3.5 g (15.8 mmol, 68%) of the title compound as a solid. Purity 100%.
¹H NMR (400 MHz, CHLOROFORM-d) δ ppm 4.72 (s, 2 H), 7.54 - 7.62 (m, 2 H), 7.70 - 7.79 (m, 1 H), 7.83 (d, J=8.21 Hz, 1 H), 8.08 (d, J=8.21 Hz, 1 H), 8.19 (d, J=8.60 Hz, 1 H).
HPLC/MS (9 min) retention time 5.45 min.
LRMS: *m*/*z* 224 (M+1).

### PREPARATION 31

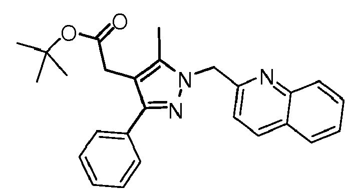

### tert-Butyl [5-methyl-3-phenyl-1-(quinolin-2-ylmethyl)-1H-pyrazol-4-yl]acetate

The title compound of Preparation 18 (80 mg, 0.29 mmol) was reacted with sodium hydride (60% dispersion in oil, 15 mg, 0.38 mmol) and with the title compound of Preparation 30 (78 mg, 0.35 mmol) according to the method of Preparation 22. The residue was purified using the SP1 Purification System (ethyl acetate-hexane gradient, 0:100 rising to 15:85) to give 35 mg (0.085 mmol, 29%) of the title compound as an oil. Purity 99%.
¹H NMR (400 MHz, CHLOROFORM-d) δ ppm 1.40 (s, 9 H), 2.22 (s, 3 H), 3.46 (s, 2 H), 5.67 (s, 2 H), 7.02 (d, J=8.60 Hz, 1 H), 7.36 (t, 1 H), 7.44 (t, J=7.42 Hz, 2 H), 7.55 (t, J=7.23 Hz, 1 H), 7.69 - 7.74 (m, 2 H), 7.73 - 7.78 (m, 1 H), 7.80 (d, J=8.21 Hz, 1 H), 8.08 (dd, J=8.21, 3.91 Hz, 2 H).
HPLC/MS (30 min) retention time 16.75 min.
LRMS: *m*/*z* 414 (M+1).

### PREPARATION 32

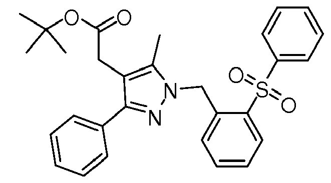

### tert-Butyl {5-methyl-3-phenyl-1-[2-(phenylsulfonyl)benzyl]-1H-pyrazol-4-yl}-acetate

The title compound of Preparation 18 (80 mg, 0.29 mmol) was reacted with sodium hydride (60% dispersion in oil, 15 mg, 0.38 mmol) and the title compound of Preparation 27 (78 mg, 0.35 mmol) according to the method of Preparation 22. The residue was purified using the SP1 Purification System (ethyl acetate-hexane gradient, 0:100 rising to 15:85) to give 100 mg (0.19 mmol, 68%) of the title compound as an oil. Purity 100%.
¹H NMR (400 MHz, CHLOROFORM-d) δ ppm 1.39 (s, 9 H), 1.95 (s, 3 H), 3.43 (s, 2 H), 5.62 (s, 2 H), 6.51 - 6.57 (m, 1 H), 7.30 - 7.36 (m, 1 H), 7.40 (t, J=7.42 Hz, 2 H), 7.44 - 7.51 (m, 2 H), 7.55 (t, J=7.42 Hz, 2 H), 7.63 (t, 3 H), 7.92 (d, J=7.42 Hz, 2 H), 8.24 - 8.29 (m, 1 H).
HPLC/MS (30 min) retention time 17.40 min.
LRMS: *m*/*z* 503 (M+1).

### PREPARATION 33

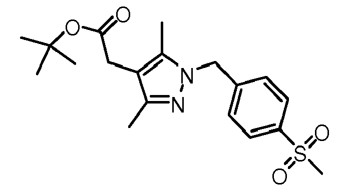

### tert-Butyl {3,5-dimethyl-1-[4-(methylsulfonyl)benzyl]-1H-pyrazol-4-yl}acetate

The title compound of Preparation 2 (80 mg, 0.38 mmol) was reacted with sodium hydride (60% dispersion in oil, 20 mg, 0.50 mmol) and 1-(bromomethyl)-4-(methylsulfonyl)benzene (114 mg, 0.46 mmol) according to the method of Preparation 22. The residue was purified using the SP1 Purification System (ethyl acetate-hexane gradient, 0:100 rising to 60:40) to give 76 mg (0.20 mmol, 53%) of the title compound as an oil. Purity 99%.
¹H NMR (400 MHz, CHLOROFORM-d) δ ppm 1.43 (s, 9 H), 2.13 (s, 3 H), 2.23 (s, 3 H), 3.03 (s, 3 H), 3.28 (s, 2 H), 5.31 (s, 2 H), 7.20 (d, J=8.21 Hz, 2 H), 7.88 (d, J=8.21 Hz, 2 H).
HPLC/MS (9 min) retention time 5.82 min.
LRMS: *m*/*z* 379 (M+1).

### PREPARATION 34

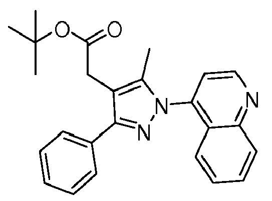

### tert-Butyl (5-methyl-3-phenyl-1-quinolin-4-yl-1H-pyrazol-4-yl)acetate

The title compound of Preparation 18 (100 mg, 0.37 mmol) was reacted with quinolin-4-ylboronic acid (127 mg, 0.73 mmol), copper (II) acetate (100 mg, 0.55 mmol) and pyridine (60 µl, 0.74 mmol) in 5 ml dichlorometane with 4Å molecular sieves. The mixture was stirred at room temperature with a stream of air bubbled through for 9 days. The mixture was filtered through celite and the combined organics were evaporated. The resulting residue was purified by reverse-phase chromatography using the SP1 Purification System to give 39 mg (0.097 mmol, 27%) of the title compound as an orange oil. Purity 100%.
¹H NMR (400 MHz, CHLOROFORM-d) δ ppm 1.47 (s, 9 H), 2.17 (s, 3 H), 3.60 (s, 2 H), 7.35 - 7.40 (m, 1 H), 7.44 (t, J=7.42 Hz, 2 H), 7.56 (d, J=8.21 Hz, 1 H), 7.66 - 7.78 (m, 5 H), 8.10 (d, J=7.82 Hz, 1 H).
HPLC/MS (9 min) retention time 6.93 min.
LRMS: *m*/*z* 400 (M+1).

### PREPARATION 35

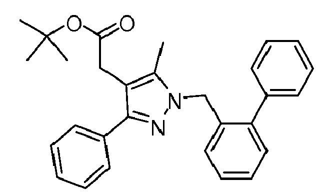

### tert-Butyl [1-(biphenyl-2-ylmethyl)-5-methyl-3-phenyl-1H-pyrazol-4-yl]acetate

The title compound of Preparation 18 (100 mg, 0.37 mmol) was reacted with sodium hydride (60% dispersion in oil, 19 mg, 0.48 mmol) and 2-(bromomethyl)biphenyl (80 µl, 0.44 mmol) according to the method of Preparation 22. The residue was purified using the SP1 Purification System (ethyl acetate-hexane gradient, 0:100 rising to 10:90) to give 100 mg (0.23 mmol, 62%) of the title compound as an oil. Purity 100%.
¹H NMR (400 MHz, CHLOROFORM-d) δ ppm 1.38 (s, 9 H), 1.95 (s, 3 H), 3.41 (s, 2 H), 5.28 (s, 2 H), 6.81 (d, J=7.82 Hz, 1 H), 7.21 - 7.48 (m, 11 H), 7.62 - 7.73 (m, 2 H).
HPLC/MS (9 min) retention time 7.75 min.
LRMS: *m*/*z* 439 (M+1).

### PREPARATION 36

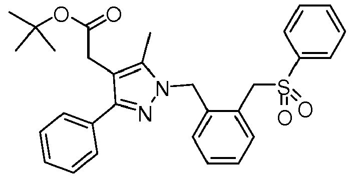

### tert-Butyl (5-methyl-3-phenyl-1-{2-[(phenylsulfonyl)methyl]benzyl}-1H-pyrazol-4-yl)acetate

The title compound of Preparation 18 (100 mg, 0.37 mmol) was reacted with sodium hydride (60% dispersion in oil, 19 mg, 0.48 mmol) and 1-(bromomethyl)-2-(phenylsulfonylmethyl)benzene (147 mg, 0.44 mmol) according to the method of Preparation 22. The residue was purified using the SP1 Purification System (ethyl acetate-hexane gradient, 0:100 rising to 50:50) to give 78 mg (0.15 mmol, 41 %) of the title compound as an oil. Purity 100%.
¹H NMR (400 MHz, CHLOROFORM-d) δ ppm 1.40 (s, 9 H), 2.20 (s, 3 H), 3.44 (s, 2 H), 4.62 (s, 2 H), 5.46 (s, 2 H), 6.80 (d, J=7.82 Hz, 1 H), 7.00 - 7.04 (m, 1 H), 7.15 - 7.20 (m, 1 H), 7.23 - 7.34 (m, 2 H), 7.36 - 7.41 (m, 2 H), 7.46 - 7.52 (m, 2 H), 7.60 - 7.65 (m, 3 H), 7.80 - 7.85 (m, 2 H).
HPLC/MS (9 min) retention time 7.25 min.
LRMS: *m*/*z* 517 (M+1)

### PREPARATION 37

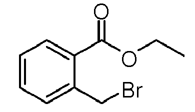

### Ethyl 2-(bromomethyl)benzoate

Ethyl 2-methylbenzoate (2.0 g, 12.2 mmol) was dissolved in 30 ml carbon tetrachloride. N-bromosuccinimide (2.17 g, 12.2 mmol) and benzoyl peroxide (80 mg, 0.33 mmol) were added and the mixture was heated at 80ºC for 4 h. The mixture was allowed to cool and was stirred at room temperature overnight. The mixture was filtered and the solvent was evaporated to give 3.0 g (11.8 mmol, 97%) of the title compound as a yellow oil. Purity 96%.
¹H NMR (400 MHz, CHLOROFORM-d) δ ppm 1.43 (t, J=7.23 Hz, 3 H), 4.42 (q, J=7.29 Hz, 2 H), 4.96 (s, 2 H), 7.38 (t, J=7.42 Hz, 1 H), 7.44 - 7.55 (m, 2 H), 7.97 (d, J=7.82 Hz, 1 H).
HPLC/MS (9 min) retention time 6.30 min.
LRMS: *m*/*z* 243 (M+1).

### PREPARATION 38

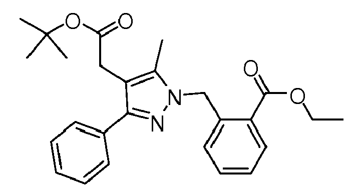

### Ethyl 2-{[4-(2-tert-butoxy-2-oxoethyl)-5-methyl-3-phenyl-1H-pyrazol-1-yl]methyl}-benzoate

The title compound of Preparation 18 (0.50 g, 1.84 mmol) was reacted with sodium hydride (60% dispersion in oil, 95 mg, 2.39 mmol) and the title compound of Preparation 37 (535.58 mg, 2.2 mmol) according to the method of Preparation 22. The residue was purified using the SP1 Purification System (ethyl acetate-hexane gradient, 0:100 rising to 10:90) to give 0.53 g (1.16 mmol, 64%) of the title compound as a pale yellow oil. Purity 96%.
¹H NMR (400 MHz, CHLOROFORM-d) δ ppm 1.40 - 1.45 (m, 12 H), 2.18 (s, 3 H), 3.49 (s, 2 H), 4.41 (q, J=7.03 Hz, 2 H), 5.81 (s, 2 H), 6.46 (d, J=7.82 Hz, 1 H), 7.28 - 7.44 (m, 5 H), 7.68 - 7.72 (m, 2 H), 8.04 (dd, J=7.64, 1.37 Hz, 1 H). HPLC/MS (9 min) retention time 7.62 min.
LRMS: *m*/*z* 435 (M+1)

### PREPARATION 39

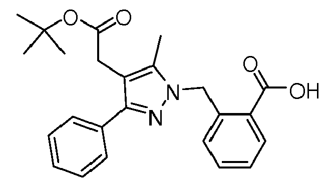

### 2-{[4-(2-tert-Butoxy-2-oxoethyl)-5-methyl-3-phenyl-1H-pyrazol-1-yl]methyl}-benzoic acid

The title compound of Preparation 38 (0.53 g, 1.167 mmol) was reacted with lithium hydroxide monohydrate (245 mg, 5.84 mmol) according to the method of Preparation 23 to give 455 mg (1.01 mmol, 87%) of the title compound as a white solid. Purity 91%.
HPLC/MS (9 min) retention time 6.98 min.
LRMS: *m*/*z* 407 (M+1)

### PREPARATION 40

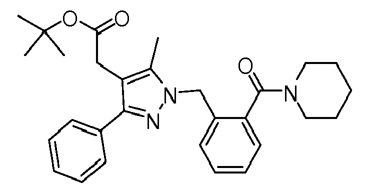

### tert-Butyl {5-methyl-3-phenyl-1-[2-(piperidin-1-ylcarbonyl)benzyl]-1H-pyrazol-4-yl}acetate

The title compound of Preparation 39 (75 mg, 0.18 mmol) was reacted with O-(7-azabenzotriazol-1-yl)-N,N,N',N'-tetramethyluronium hexafluorophosphate (HATU) (67 mg, 0.18 mmol), diisopropylethylamine (68 µl, 0.39 mmol) and piperidine (21 µl, 0.21 mmol) according to the method of Preparation 24. The residue was purified using the SP1 Purification System (ethyl acetate-hexane gradient, 0:100 rising to 25:75) to give 17 mg (0.036 mmol, 20%) of the title compound as an oil. Purity 98%.
¹H NMR (400 MHz, CHLOROFORM-d) δ ppm 1.40 (s, 9 H), 1.52 - 1.74 (m, 6 H), 2.22 (s, 3 H), 3.06 (br. s., 1 H), 3.20 (br. s., 1 H), 3.44 (s, 2 H), 3.50 (br. s., 1 H), 3.88 (br. s., 1 H), 5.36 (br. s., 2 H), 6.86 - 6.93 (m, 1 H), 7.15 - 7.23 (m, 1 H), 7.23 - 7.35 (m, 3 H), 7.39 (t, J=7.42 Hz, 2 H), 7.64 (d, J=7.42 Hz, 2 H).
HPLC/MS (309 min) retention time 17.20 min.
LRMS: *m*/*z* 474 (M+1).

### PREPARATION 41

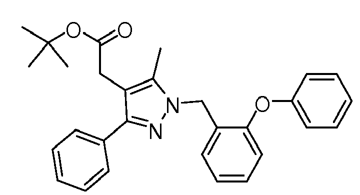

### tert-Butyl [5-methyl-1-(2-phenoxybenzyl)-3-phenyl-1H-pyrazol-4-yl]acetate

The title compound of Preparation 18 (100 mg, 0.37 mmol) was reacted with sodium hydride (60% dispersion in oil, 19 mg, 0.48 mmol) and 1-(bromomethyl)-2-phenoxybenzene (130 mg, 0.44 mmol) according to the method of Preparation 22. The residue was purified using the SP1 Purification System (ethyl acetate-hexane gradient, 0:100 rising to 50:50) to give 123 mg (0.27 mmol, 74%) of the title compound as an oil. Purity 100%.
¹H NMR (400 MHz, CHLOROFORM-d) δ ppm 1.41 (s, 9 H), 2.20 (s, 3 H), 3.43 (s, 2 H), 5.41 (s, 2 H), 6.82 - 6.90 (m, 2 H), 6.96 - 7.06 (m, 3 H), 7.07 - 7.13 (m, 1 H), 7.18 - 7.24 (m, 1 H), 7.29 - 7.36 (m, 3 H), 7.38 - 7.44 (m, 2 H), 7.66 - 7.70 (m, 2 H).
HPLC/MS (9 min) retention time 7.80 min.
LRMS: *m*/*z* 455 (M+1)

### PREPARATION 42

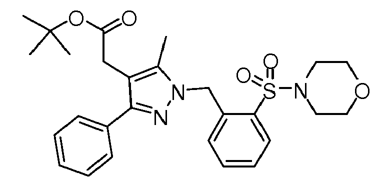

### tert-Butyl {5-methyl-1-[2-(morpholin-4-ylsulfonyl)benzyl]-3-phenyl-1H-pyrazol-4-yl}acetate

The title compound of Preparation 18 (100 mg, 0.37 mmol) was reacted with sodium hydride (60% dispersion in oil, 19 mg, 0.48 mmol) and 4-(2-(bromomethyl)phenylsulfonyl)morpholine (157 mg, 0.44 mmol) according to the method of Preparation 22. The residue was purified using the SP1 Purification System (ethyl acetate-hexane gradient, 0:100 rising to 50:50) to give 177 mg (0.35 mmol, 94%) of the title compound as an oil. Purity 100%.
¹H NMR (400 MHz, CHLOROFORM-d) δ ppm 1.38 (s, 9 H), 2.14 (s, 3 H), 3.16 - 3.20 (m, 4 H), 3.46 (s, 2 H), 3.70 - 3.76 (m, 4 H), 5.77 (s, 2 H), 6.62 (d, J=7.42 Hz, 1 H), 7.28 - 7.34 (m, 1 H), 7.36 - 7.48 (m, 4 H), 7.64 - 7.68 (m, 2 H), 7.92 (dd, J=7.82, 1.56 Hz, 1 H).
HPLC/MS (9 min) retention time 7.18 min.
LRMS: *m*/*z* 512 (M+1)

### PREPARATION 43

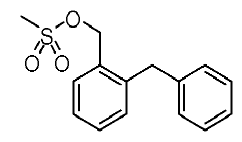

### 2-Benzylbenzyl methanesulfonate

(2-Benzylphenyl)methanol (0.30 g, 1.51 mmol) was dissolved in 20 ml dichlormethane. Triethylamine (275 µl, 1.97 mmol) and methanesulfonyl chloride (140 µl, 1.82 mmol) were added and the mixture was agitated at room temperature overnight. A further 275 µl triethylamine and 140 µl methanesulfonyl chloride were added and the mixture was stirred at room temperature 2 h. The mixture was concentrated under reduced pressure and the residue was partitioned between water and ethyl acetate. The organic layer was washed three times with 4% w/v sodium bicarbonate solution and dried over magnesium sulphate. Filtration and evaporation give 285 mg of the crude title compound as a yellow oil. Purity 74%.
HPLC/MS (9 min) retention time 7.23 min.
LRMS: *m*/*z* 277 (M+1)

### PREPARATION 44

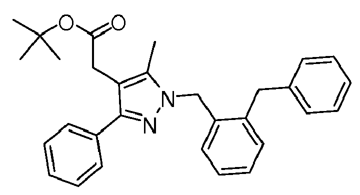

### tert-Butyl [1-(2-benzylbenzyl)-5-methyl-3-phenyl-1H-pyrazo)-4-yl]acetate

The title compound of Preparation 18 (100 mg, 0.37 mmol) was reacted with sodium hydride (60% dispersion in oil, 19 mg, 0.48 mmol) and the crude title compound of Preparation 43 (165 mg) according to the method of Preparation 22. The residue was purified using the SP1 Purification System (ethyl acetate-hexane gradient, 0:100 rising to 40:60) to give 105 mg (0.23 mmol, 63%) of the title compound as an oil. Purity 100%.
¹H NMR (400 MHz, CHLOROFORM-d) δ ppm 1.39 (s, 9 H), 1.94 (s, 3 H), 3.42 (s, 2 H), 4.10 (s, 2 H), 5.24 (s, 2 H), 6.54 (d, J=7.82 Hz, 1 H), 7.11 - 7.17 (m, 3 H), 7.17 - 7.23 (m, 3 H), 7.24 - 7.34 (m, 3 H), 7.36 - 7.43 (m, 2 H), 7.64 - 7.69 (m, 2 H).
HPLC/MS (9 min) retention time 7.85 min.
LRMS:m/z 453 (M+1)

### PREPARATION 45

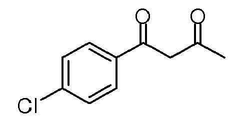

### 1-(4-Chlorophenyl)butane-1,3-dione

Sodium hydride (60% dispersion in oil, 1.03 g, 25.7 mmol) was first washed with hexanes to remove the oil and was then suspended in 10 ml tetrahydrofuran an cooled to 0 ºC in an ice-bath. A solution of 1-(4-chlorophenyl)ethanone (2.0 g, 13 mmol) in ethyl acetate (2.5 ml, 26 mmol) was added drop-wise and the mixture was stirred at room temperature overnight. The mixture was then heated at 40ºC for 26 h. The mixture was allowed to cool and was diluted with water. The mixture was acidified with 2N hydrochloric acid and was extracted with ethyl acetate. The combined organics were washed with water, brine and dried over sodium sulphate. The residue was purified using the SP1 Purification System (ethyl acetate-hexane gradient, 0:100 rising to 10:90) to give 1.28 g of the crude title compound as a yellow solid. Purity 85%.
¹H NMR (400 MHz, CHLOROFORM-d) δ ppm 2.21 (s, 3 H), 6.14 (s, 1 H), 7.42 (d, J=8.60 Hz, 2 H), 7.82 (d, J=8.60 Hz, 2 H).
HPLC/MS (9 min) retention time 6.58 min.
LRMS: *m*/*z* 197 (M+1).

### PREPARATION 46

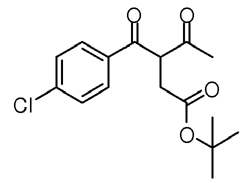

### tert-Butyl 3-(4-chlorobenzoyl)-4-oxopentanoate

The crude title compound of Preparation 45 (1.28 g) was reacted with sodium hydride (60% dispersion in oil, 330 mg, 8.25 mmol) and tert-butyl 2-bromoacetate (1 ml, 6.6 mmol) according to the method of Preparation 1 to give 1.95 g of the crude title compound as an oil. Used as such without further purification. Purity 84%.
¹H NMR (400 MHz, CHLOROFORM-d) δ ppm 1.40 (s, 9 H), 2.16 (s, 3 H), 2.92 (d, J=7.03 Hz, 2 H), 4.87 (t, J=7.03 Hz, 1 H), 7.48 (d, J=8.60 Hz, 2 H), 7.97 (d, J=8.60 Hz, 2 H).
HPLC/MS (9 min) retention time 6.65 min.
LRMS: *m*/*z* 313 (M+1).

### PREPARATION 47

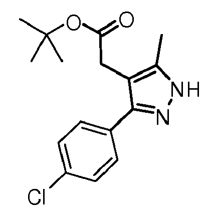

### tert-Butyl [5-(4-chlorophenyl)-3-methyl-1H-pyrazol-4-yl]acetate

The crude title compound of Preparation 46 (1.90 g) was reacted with hydrazine monohydrate (0.38 ml, 7.7 mmol) according to the method of Preparation 2. The residue was purified using the SP1 Purification System (ethyl acetate-hexane gradient, 0:100 rising to 25:75) to give 1.30 g (4.19 mmol, 32% over three steps) of the title compound as a yellow oil. Purity 99%.
¹H NMR (400 MHz, CHLOROFORM-d) δ ppm 1.42 (s, 9 H), 2.32 (s, 3 H), 3.39 (s, 2 H), 7.40 (d, J=8.21 Hz, 2 H), 7.56 (d, J=8.60 Hz, 2 H).
HPLC/MS (9 min) retention time 6.75 min.
LRMS: *m*/*z* 307 (M+1).

### PREPARATION 48

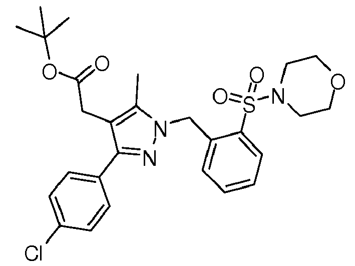

### tert-Butyl {3-(4-chlorophenyl)-5-methyl-1-[2-(morpholin-4-ylsulfonyl)benzyl]-1H-pyrazol-4-yl}acetate

The title compound of Preparation 47 (100 mg, 0.32 mmol) was reacted with sodium hydride (60% dispersion in oil, 17 mg, 0.43 mmol) and 4-(2-(bromomethyl)-phenylsulfonyl)morpholine (123 mg, 0.38 mmol) according to the method of Preparation 22. The residue was purified using the SP1 Purification System (ethyl acetate-hexane gradient, 0:100 rising to 15:85) to give 115 mg (0.21 mmol, 65%) of the title compound as a colourless oil. Purity 99%.
¹H NMR (400 MHz, CHLOROFORM-d) δ ppm 1.42 (s, 9 H), 2.17 (s, 3 H), 3.22 (t, 4 H), 3.47 (br. s., 2 H), 3.76 (t, 4 H), 5.79 (s, 2 H), 6.63 (d, J=7.82 Hz, 1 H), 7.37 - 7.51 (m, 4 H), 7.65 (d, J=8.21 Hz, 2 H), 7.95 (d, J=7.82 Hz, 1 H).
HPLC/MS (30 min) retention time 18.05 min.
LRMS: *m*/*z* 546 (M+1).

### PREPARATION 49

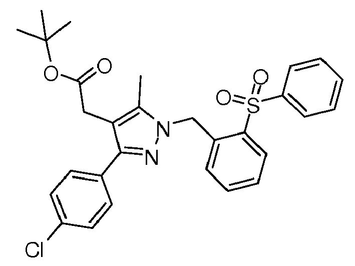

### tert-butyl {3-(4-chlorophenyl)-5-methyl-1-[2-(phenylsulfonyl)benzyl]-1H-pyrazol-4-yl}acetate

The title compound of Preparation 47 (100 mg, 0.32 mmol) was reacted with sodium hydride (60% dispersion in oil, 17 mg, 0.43 mmol) and the title compound of the Preparation 27 (134 mg, 0.39 mmol) according to the method of Preparation 22. The residue was purified using the SP1 Purification System (ethyl acetate-hexane gradient, 0:100 rising to 9:91) to give 170 mg (0.32 mmol, 98%) of the title compound as a pale yellow oil. Purity 100%.
¹H NMR (400 MHz, CHLOROFORM-d) δ ppm 1.40 (s, 9 H), 1.95 (s, 3 H), 3.40 (s, 2 H), 5.61 (s, 2 H), 6.50 - 6.55 (m, 1 H), 7.37 (d, J=8.60 Hz, 2 H), 7.44 - 7.50 (m, 2 H), 7.51 - 7.65 (m, 5 H), 7.92 (d, J=7.42 Hz, 2 H), 8.21 - 8.28 (m, 1 H).
HPLC/MS (30 min) retention time 18.47 min.
LRMS: *m*/*z* 537 (M+1).

### PREPARATION 50

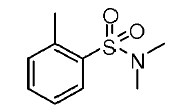

### N,N,2-Trimethylbenzenesulfonamide

2-Methylbenzene-1-sulfonyl chloride (1.0 g, 5.25 mmol) was dissolved in 6.5 ml tetrahydrofuran. A solution of dimethylamine in tetrahydrofuran (2M, 6.6 ml, 13.2 mmol) was added drop-wise and with stirring. Upon addition, the mixture was stirred for 10 min at room temperature. The mixture was filtered and was concentrated under reduced pressure to give 1.04 g (5.19 mmol, 99%) of the title compound. Used as such without further purification. Purity 100%.
¹H NMR (400 MHz, CHLOROFORM-d) δ ppm 2.6 (s, 3 H), 2.8 (s, 6 H), 7.3 (m, 2 H), 7.5 (t, J=7.4 Hz, 1 H), 7.9 (m, 1 H).
HPLC/MS (15 min) retention time 6.23 min.
LRMS: *m*/*z* 199 (M+1).

### PREPARATION 51

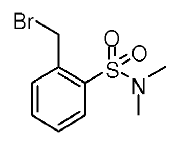

### 2-(Bromomethyl)-N,N-dimethylbenzenesulfonamide

The title compound of Preparation 50 (0.40 g, 2.0 mmol) was dissolved in 13 ml carbon tetrachloride. N-Bromosuccinimide (0.39 mg, 2.2 mmol) was added and the mixture was heated at 90ºC for 5 min. 2,2'-Azobis(2-methylpropionitrile) (100 mg, 0.60 mmol) was then added and the mixture was heated at 90ºC 2 h. The mixture was allowed to cool and was filtered. The filtrate was concentrated under reduced pressure to give 190 mg of the crude title compound as a yellow oil. Purity 81%.
HPLC/MS (9 min) retention time 5.58 min.
LRMS: *m*/*z* 278 (M+1).

### PREPARATION 52

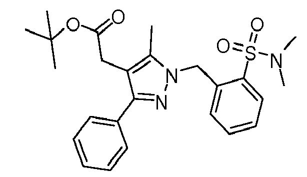

### tert-Butyl (1-{2-[(dimethylamino)sulfonyl]benzyl}-5-methyl-3-phenyl-1H-pyrazol-4-yl)acetate

The title compound of Preparation 18 (125 mg, 0.46 mmol) was reacted with sodium hydride (60% dispersion in oil, 24 mg, 0.6 mmol) and the crude title compound of the Preparation 51 (190 mg) according to the method of Preparation 22. The residue was purified using the SP1 Purification System (ethyl acetate-hexane gradient, 0:100 rising to 30:70) to give 83 mg (0.17 mmol, 37%) of the title compound as a colourless oil. Purity 97%.
¹H NMR (400 MHz, CHLOROFORM-d) δ ppm 1.41 (s, 9 H), 2.16 (s, 3 H), 2.85 (s, 6 H), 3.48 (s, 2 H), 5.80 (s, 2 H), 6.61 (d, J=7.42 Hz, 1 H), 7.30 - 7.39 (m, 1 H), 7.39 - 7.49 (m, 4 H), 7.69 (d, J=7.42 Hz, 2 H), 7.95 (d, J=6.64 Hz, 1 H).
HPLC/MS (30 min) retention time 16.90 min.
LRMS: *m*/*z* 470 (M+1).

### PREPARATION 53

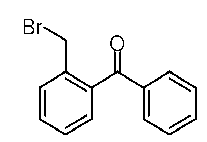

### [2-(Bromomethyl)phenyl](phenyl)methanone

Phenyl (*o*-tolyl)methanone (1.0 g, 5.1 mmol) was reacted with N-bromosuccinimide (0.98 g, 5.51 mmol) and 2,2'-azobis(2-methylpropionitrile) (0.25 g, 1.52 mmol) according to the method of Preparation 51. The residue was purified using the SP1 Purification System (ethyl acetate-hexane gradient, 0:100 rising to 20:80) to give 0.44 g of the crude title compound as a green oil. Purity 31%.
¹H NMR (400 MHz, CHLOROFORM-d) δ ppm 1.40 (s, 9 H), 2.17 (s, 3 H), 3.40 (s, 2 H), 5.54 (s, 2 H), 6.82 - 6.87 (m, 1 H), 7.28 - 7.35 (m, 2 H), 7.35 - 7.49 (m, 6 H), 7.56 - 7.65 (m, 3 H), 7.78 - 7.83 (m, 2 H).
HPLC/MS (9 min) retention time 7.60 min.
LRMS: *m*/*z* 467 (M+1).

### PREPARATION 54

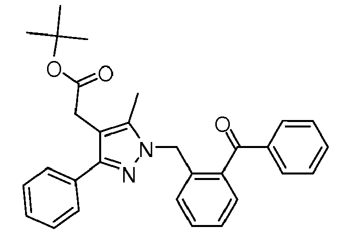

### tert-Butyl [1-(2-benzoyl benzyl)-5-methyl-3-phenyl-1H-pyrazol-4-yl]acetate

The title compound of Preparation 18 (275 mg, 1.01 mmol) was reacted with sodium hydride (60% dispersion in oil, 52.51 mg, 1.31 mmol) and the crude title compound of the Preparation 53 (0.44 g) according to the method of Preparation 22. The residue was purified using the SP1 Purification System (ethyl acetate-hexane gradient, 0:100 rising to 20:80) to give 208 mg (0.44 mmol, 44%) of the title compound as an oil. Purity 100%.
¹H NMR (400 MHz, CHLOROFORM-d) δ ppm 1.40 (s, 9 H), 2.17 (s, 3 H), 3.40 (s, 2 H), 5.54 (s, 2 H), 6.82 - 6.87 (m, 1 H), 7.28 - 7.35 (m, 2 H), 7.35 - 7.49 (m, 6 H), 7.56 - 7.65 (m, 3 H), 7.78 - 7.83 (m, 2 H).
HPLC/MS (9 min) retention time 7.60 min.
LRMS: *m*/*z* 467 (M+1).

### PREPARATION 55

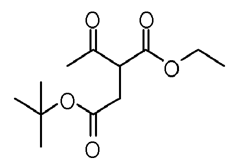

### 4-tert-Butyl 1-ethyl 2-acetylsuccinate

Ethyl 3-oxobutanoate (3.0 g, 23 mmol) was reacted with tert-butyl 2-bromoacetate (4.0 ml, 27.7 mmol) and sodium hydride (60% dispersion in oil, 1.48 g, 37 mmol) according to the method of Preparation 1 to give 5.46 g of the crude title compound as a colourless oil. Used as such without further purification. Purity 79%
HPLC/MS (9 min) retention time 5.92 min.
LRMS: *m*/*z* 245 (M+1).

### PREPARATION 56

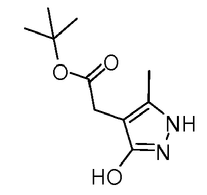

### tert-Butyl (3-hydroxy-5-methyl-1H-pyrazol-4-yl)acetate

The crude title compound of Preparation 55 was dissolved in 100 ml acetic acid. Sodium acetate (7.57 g, 92.3 mmol) and hydrazine monohydrate (0.90 ml, 18.5 mmol) were added and the mixture was heated at 70ºC overnight. The mixture was diluted with water. The aqueous layer was extracted three times with ethyl acetate. The combined organics were washed with brine, potassium carbonate diluted solution and brine. The organic layer was dried over magnesium sulphate, filtered and evaporated. The residue was treated with acetonitrile to give 2.22 g (10.5 mmol, 46% over two steps) of the title compound as a white solid. Purity 100%
¹H NMR (400 MHz, DMSO-d6) δ ppm 1.38 (s, 9 H), 2.03 (s, 3 H), 3.12 (s, 2 H), 9.52 (br. s., 1 H), 11.13 (br. s., 1 H)
HPLC/MS (9 min) retention time 4.37 min.
LRMS: *m*/*z* 213 (M+1).

### PREPARATION 57

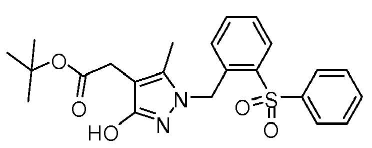

### tert-Butyl {3-hydroxy-5-methyl-1-[2-(phenylsulfonyl)benzyl]-1H-pyrazol-4-yl}-acetate

The title compound of Preparation 56 (100 mg, 0.47 mmol) was reacted with sodium hydride (60% dispersion in oil, 24.5 mg, 0.61 mmol) and the title compound of the Preparation 27 (175 mg, 0.57 mmol) according to the method of Preparation 22. The residue was purified by reverse-phase chromatography using the SP1 Purification System to give 106 mg (0.23 mmol, 51%) of the title compound. Purity 100%.
¹H NMR (400 MHz, CHLOROFORM-d) δ ppm 1.40 (s, 9 H), 2.13 (s, 3 H), 3.20 (s, 2 H), 5.59 (s, 2 H), 7.40 - 7.53 (m, 4 H), 7.54 - 7.60 (m, 1 H), 7.76 (d, J=7.82 Hz, 1 H), 7.86 - 7.91 (m, 2 H), 8.17 (dd, J=7.82 Hz, 1 H).
HPLC/MS (9 min) retention time 6.75 min.
LRMS: *m*/*z* 443 (M+1).

### PREPARATION 58

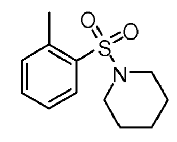

### 1-[(2-Methylphenyl)sulfonyl]piperidine

Piperidine (250 mg, 2.94 mmol) and triethylamine (0.82 ml, 5.87 mmol) were dissolved in 13 ml of dichloromethane. 2-Methylbenzene-1-sulfonyl chloride (840 mg, 4.41 mmol) in 3 ml of dichloromethane was slowly added and the mixture was stirred at room temperature 2 h. The mixture was washed sequentially with 4% w/v sodium bicarbonate solution, 2N sodium hydroxide and water. The resulting organic layer was dried over sodium sulphate, filtered and evaporated. The residue was purified using the SP1 Purification System (ethyl acetate-hexane gradient, 0:100 rising to 10:90) to give 210 mg (0.8 mmol, 28%) of the title compound as a colourless oil. Purity 92%.
¹H NMR (400 MHz, CHLOROFORM-d) δ ppm 1.48 - 1.54 (m, 2 H), 1.62 (quin, J=5.47 Hz, 4 H), 2.64 (s, 3 H), 3.14 (t, 4 H), 7.28 - 7.35 (m, 2 H), 7.40 - 7.46 (m, 1 H), 7.90 (d, J=7.82 Hz, 1 H).
HPLC/MS (9 min) retention time 6.35 min.
LRMS: *m*/*z* 240 (M+1).

### PREPARATION 59

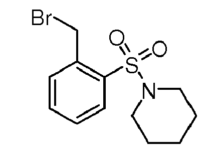

### 1-{[2-(Bromomethyl)phenyl]sulfonyl}piperidine

1-(o-Tolylsulfonyl)piperidine (200 mg, 0.84 mmol) was reacted with N-bromosuccinimide (164 mg, 0.92 mmol) and 2,2'-azobis(2-methylpropionitrile) (15 mg, 0.09 mmol) according to the method of Preparation 51. The residue was purified by column chromatography (ethyl acetate-hexane gradient, 15:85 rising to 20:80) to give 60 mg (0.13 mmol, 16%) of the title compound as a colourless oil. Purity 71%.
HPLC/MS (15 min) retention time 8.02 min.
LRMS: *m*/*z* 320 (M+1).

### PREPARATION 60

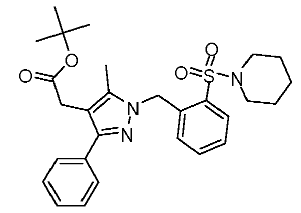

### tert-Butyl {5-methyl-3-phenyl-1-[2-(piperidin-1-ylsulfonyl)benzyl]-1H-pyrazol-4-yl}acetate

The title compound of Preparation 18 (30 mg, 0.11 mmol) was reacted with sodium hydride (60% dispersion in oil, 6 mg, 0.15 mmol) and the title compound of the Preparation 59 (59 mg, 0.13 mmol) according to the method of Preparation 22. The residue was purified by column chromatography (ethyl acetate-hexane, 10:90 rising to 28:72) to give 30 mg (0.058 mmol, 53%) of the title compound. Purity 100%.
¹H NMR (400 MHz, CHLOROFORM-d) δ ppm 1.41 (s, 9 H), 1.54 - 1.60 (m, 2 H), 1.62 - 1.72 (m, 4 H), 2.15 (s, 3 H), 3.20 (t, 4 H), 3.48 (s, 2 H), 5.80 (s, 2 H), 6.61 (d, J=7.42 Hz, 1 H), 7.32 - 7.40 (m, 2 H), 7.43 (t, 3 H), 7.70 (d, J=7.03 Hz, 2 H), 7.94 (d, J=1.56 Hz, 1 H).
HPLC/MS (9 min) retention time 7.52 min.
LRMS: *m*/*z* 510 (M+1).

### PREPARATION 61

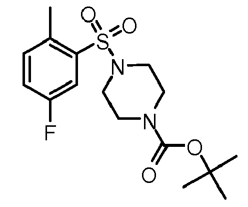

### tert-Butyl 4-[(5-fluoro-2-methylphenyl)sulfonyl]piperazine-1-carboxylate

5-Fluoro-2-methylbenzene-1-sulfonyl chloride (700 mg, 3.36 mmol) was dissolved in 5 ml tetrahydrofuran. A solution of dimethylamine (1.56 g, 8.39 mmol) in 4 ml of tetrahydrofuran was added drop-wise and with stirring. Upon addition, the mixture was stirred for 30 minutes at room temperature. The mixture was filtered and was concentrated. The residue was taken up in dichloromethane and washed sequentially with 2H hydrochloric acid, water and brine. The resulting organic layer was dried over sodium sulphate, filtered and evaporated to give 1.2 g (3.31 mmol, 99%) of the title compound. Purity 99%.
¹H NMR (400 MHz, CHLOROFORM-d) δ ppm 1.44 (s, 9 H), 2.59 (s, 3 H), 3.16 (t, 4 H), 3.49 (t, 4 H), 7.14 - 7.22 (m,1 H), 7.30 (dd, J=8.40, 5.28 Hz, 1 H), 7.61 (dd, J=8.40, 2.54 Hz, 1 H).
HPLC/MS (15 min) retention time 8.38 min.
LRMS: *m*/*z* 359 (M+1).

### PREPARATION 62

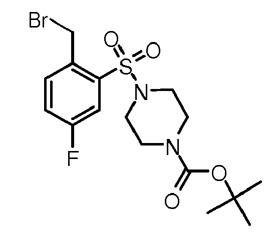

### tert-Butyl 4-{[2-(bromomethyl)-5-fluorophenyl]sulfonyl}piperazine-1-carboxylate

The title compound of Preparation 61 (1.2 g, 3.31 mmol) was reacted with N-bromosuccinimide (655 mg, 3.68 mmol) and 2,2'-azobis(2-methylpropionitrile) (73 mg, 0.44 mmol) according to the method of Preparation 51. The residue was purified by column chromatography (ethyl acetate-hexane, 10:90 rising to 37:63) to give 39 mg (0.086 mmol, 3%) of the title compound as a solid. Purity 97%.
HPLC/MS (15 min) retention time 8.65 min.
LRMS: *m*/*z* 438 (M+1).

### PREPARATION 63

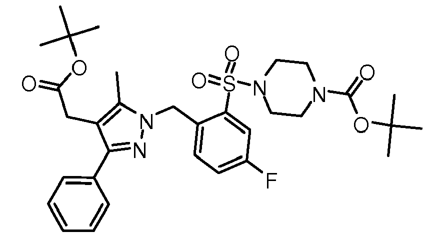

### tert-Butyl 4-[(2-{[4-(2-tert-butoxy-2-oxoethyl)-5-methyl-3-phenyl-1H-pyrazol-1-yl]-methyl}-5-fluorophenyl)sulfonyl]piperazine-1-carboxylate

The title compound of Preparation 18 (22 mg, 0.08 mmol) was reacted with sodium hydride (60% dispersion in oil, 4 mg, 0.09 mmol) and the title compound of the Preparation P062 (39 mg, 0.08 mmol) according to the method of Preparation P022. The residue was purified by column chromatography (ethyl acetate-hexane, 15:85) to give 13 mg (0.02 mmol, 26%) of the title compound. Purity 100%.
HPLC/MS (15 min) retention time 10.45 min.
LRMS: *m*/*z* 629 (M+1).

### EXAMPLES

### EXAMPLE 1

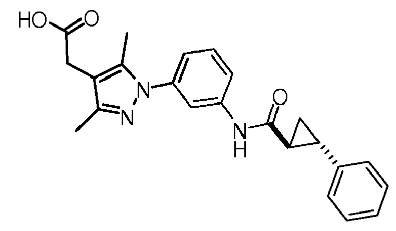

### {3,5-Dimethyl-1-[3-({[trans-2-phenylcyclopropyl]carbonyl}amino)phenyl]-1H-pyrazol-4-yl}acetic acid

The title compound of Preparation 6 (200 mg, 0.45 mmol) was dissolved in 3 ml chloroform. 2 ml Trifluoroacetic acid was added and the mixture was stirred for 2 h at room temperature. The mixture was concentrated under reduced pressure. The residue was taken up in a little water, the mixture frozen and lyophilised overnight to give 170 mg (0.42 mmol, 93%) of the title compound as a white solid. Purity 93%.
¹H NMR (400 MHz, DMSO-*d*₆) δ ppm 10.46 (1 H, s), 7.85 (1 H, s), 7.52 (1 H, d, *J*=8.2 Hz), 7.40 (1 H, t, *J*=8.0 Hz), 7.30 (2 H, t, *J*=7.4 Hz), 7.17 - 7.24 (3 H, m), 7.15 (1 H, d, *J*=7.8 Hz), 3.37 (2 H, s), 2.35 - 2.43 (1 H, m), 2.24 (3 H, s), 2.14 (3 H, s), 2.03-2.11 (1 H, m), 1.51 (1 H, dt, *J*=9.0, 4.5 Hz), 1.35 - 1.44 (1 H, m).
HPLC/MS (30 min) retention time 14.96 min.
LRMS: *m*/*z* 390 (M+1).

### EXAMPLE 2

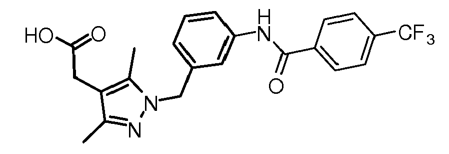

### [3,5-Dimethyl-1-(3-{[4-(trifluoromethyl)benzoyl]amino}benzyl)-1H-pyrazol-4-yl]acetic acid

The title compound of Preparation 9 (55 mg, 0.11 mmol) was dissolved in 2 ml dichloromethane. 2 ml Trifluoroacetic acid was added and the mixture was stirred for 2 h at room temperature. The mixture was concentrated under reduced pressure. The residue was tritured several times with ether, decanting each time. The residue was dried under reduced pressure to give 43 mg (0.09 mmol, 91%) of the title compound. Purity 97%.
¹H NMR (400 MHz, CHLOROFORM-d) δ ppm 8.45 (1 H, s), 8.05 (2 H, d, J=7.8 Hz), 7.89 (1 H, d, J=8.2 Hz), 7.75 (2 H, d, J=8.2 Hz), 7.46 (1 H, br. s.), 7.36 (1 H, t, J=7.8 Hz), 6.97 (1 H, d, J=7.8 Hz), 5.34 (2 H, s), 3.41 (2 H, s), 2.30 (3 H, s), 2.26 (3 H, s).
HPLC/MS (30 min) retention time 15.14 min.
LRMS: *m*/*z* 432 (M+1).

### EXAMPLE 3

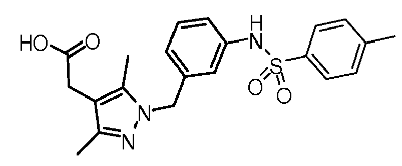

### [3,5-Dimethyl-1-(3-{[(4-methylphenyl)sulfonyl]amino}benzyl)-1H-pyrazol-4-yl]acetic acid

The title compound of Preparation 10 (20 mg, 0.042 mmol) was treated with trifluoroacetic acid according to the method of Example 2 to give 7 mg (0.017 mmol, 37%) of the title compound as a white solid. Purity 96%.
¹H NMR (400 MHz, CHLOROFORM-d) δ ppm 7.60 (2 H, d, J=8.2 Hz), 7.20 (2 H, d, J=8.2 Hz), 7.11 - 7.19 (2 H, m), 7.02 (1 H, d, J=8.2 Hz), 6.80 (1 H, d, J=7.8 Hz), 6.56 (1 H, s), 5.14 (2 H, s), 3.36 (2 H, s), 2.37 (3 H, s), 2.22 (3 H, s), 2.03 (3 H, s).
HPLC/MS (30 min) retention time 13.21 min.
LRMS: *m*/*z* 414 (M+1).

### EXAMPLE 4

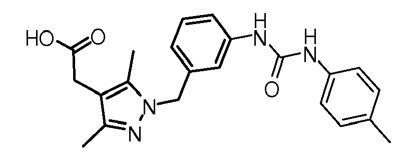

### {3,5-Dimethyl-1-[3-({[(4-methylphenyl)amino]carbonyl}amino)benzyl]-1H-pyrazol-4-yl}acetic acid

The crude title compound of Preparation 11 (155 mg) was treated with trifluoroacetic acid according to the method of Example 2, followed by purification by reverse-phase chromatography using the SP1 Purification system to give 12 mg (0.029 mmol, 18% over two steps) of the title compound as a white solid. Purity 94%.
HPLC/MS (30 min) retention time 11.32 min.
LRMS: *m*/*z* 393 (M+1).

### EXAMPLE 5

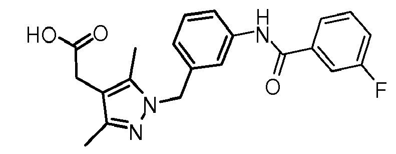

### (1-{3-[(3-Fluorobenzoyl)amino]benzyl}-3,5-dimethyl-1H-pyrazol-4-yl)acetic acid

The title compound of Preparation 12 (70 mg, 0.16 mmol) was treated with trifluoroacetic acid according to the method of Example 1 to give 52 mg (0.12 mmol, 77%) of the title compound as a white solid. Purity 95%.
¹H NMR (400 MHz, DMSO-*d*₆) δ ppm 10.35 (1 H, s), 7.80 (1 H, d, *J*=7.4 Hz), 7.76 (1 H, d, *J*=9.8 Hz), 7.70 (1 H, d, *J*=8.6 Hz), 7.61 (1 H, s), 7.54 - 7.60 (1 H, m), 7.44 (1 H, td, *J*=8.4, 2.3 Hz), 7.31 (1 H, t, *J*=7.8 Hz), 6.86 (1 H, d, *J*=7.8 Hz), 5.19 (2 H, s), 3.27 (2 H, s), 2.14 (3 H, s), 2.06 (3 H, s).
HPLC/MS (30 min) retention time 13.27 min.
LRMS: *m*/*z* 382 (M+1).

### EXAMPLE 6

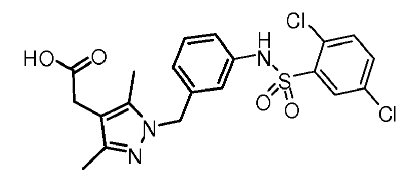

### [1-(3-{[(2,5-Dichlorophenyl)sulfonyl]amino}benzyl)-3,5-dimethyl-1H-pyrazol-4-yl]acetic acid

The title compound of Preparation 13 (40 mg, 0.074 mmol) was treated with trifluoroacetic acid according to the method of Example 1 to give 25 mg (0.05 mmol, 68%) of the title compound as a solid. Purity 98%.
¹H NMR (400 MHz, CHLOROFORM-*d*) δ ppm 7.94 (1 H, d, *J*=1.6 Hz), 7.37 - 7.44 (2 H, m), 7.33 (1 H, s), 7.19 (1 H, t, *J*=8.0 Hz), 7.05 (1 H, d, *J*=8.2 Hz), 6.87 (1 H, d, *J*=7.4 Hz), 6.72 (1 H, s), 5.20 (2 H, s), 3.41 (2 H, s), 2.25 (3 H, s), 2.09 (3 H, s).
HPLC/MS (30 min) retention time 14.49 min.
LRMS: *m*/*z* 469 (M+1).

### EXAMPLE 7

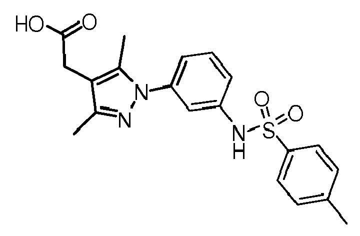

### [3,5-Dimethyl-1-(3-{[(4-methylphenyl)sulfonyl]amino}phenyl)-1H-pyrazol-4-yl]acetic acid

The title compound of Preparation 14 (90 mg, 0.20 mmol) was treated with trifluoroacetic acid according to the method of Example 1 to give 72 mg (0.176 mmol, 89%) of the title compound as a white solid. Purity 95%.
¹H NMR (400 MHz, DMSO-*d*₆) δ ppm 10.46 (1 H, s), 7.67 (2 H, d, *J*=8.2 Hz), 7.35 (2 H, d, *J*=7.8 Hz), 7.28 - 7.34 (1 H, m), 7.16 (1 H, s), 7.07 - 7.14 (2 H, m), 3.34 (2 H, s), 2.33 (3 H, s), 2.11 (6 H, s).
HPLC/MS (30 min) retention time 13.68 min.
LRMS: *m*/*z* 400 (M+1).

### EXAMPLE 8

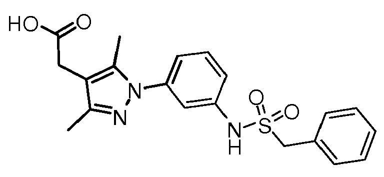

### (1-{3-[(Benzylsulfonyl)amino]phenyl}-3,5-dimethyl-1H-pyrazol-4-yl)acetic acid

The crude title compound of Preparation 15 (100 mg, 0.18 mmol) was dissolved in 2 ml chloroform. 2 ml Trifluoroacetic acid was added and the mixture was stirred for 2 h at room temperature. The mixture was concentrated under reduced pressure. The residue was tritured several times with ether, decanting each time. The residue was dried under reduced pressure to give 5 mg (0.012 mmol, 3% over two steps) of the title compound. Purity 88%.
¹H NMR (400 MHz, CHLOROFORM-*d*) δ ppm 7.26 - 7.42 (6 H, m), 7.16 - 7.22 (2 H, m), 7.11 (1 H, d, *J*=8.2 Hz), 4.35 (2 H, s), 3.43 (2 H, s), 2.25 (6 H, s).
HPLC/MS (30 min) retention time 13.19 min.
LRMS: *m*/*z* 400 (M+1).

### EXAMPLE 9

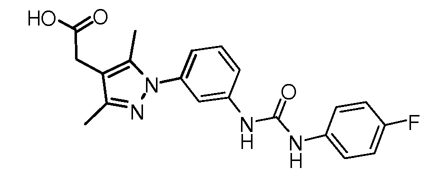

### {1-[3-({[(4-Fluorophenyl)amino]carbonyl}amino)phenyl]-3,5-dimethyl-1H-pyrazol-4-yl}acetic acid

The title compound of Preparation 16 (140 mg, 0.32 mmol) was treated with trifluoroacetic acid according to the method of Example 1 to give 103 mg (0.25 mmol, 84%) of the title compound as a white solid. Purity 93%.
¹H NMR (400 MHz, DMSO-*d*₆) δ ppm 12.26 (1 H, br. s.), 8.92 (1 H, s), 8.79 (1 H, s), 7.73 (1 H, s), 7.47 (2 H, dd, *J*=9.0, 4.7 Hz), 7.38 (1 H, t, *J*=7.8 Hz), 7.29 - 7.34 (1 H, m), 7.13 (2 H, t, *J*=9.0 Hz), 7.07 (1 H, d, *J*=8.2 Hz), 2.24 (3 H, s), 2.14 (3 H, s).
HPLC/MS (30 min) retention time 13.81 min.
LRMS: *m*/*z* 383 (M+1).

### EXAMPLE 10

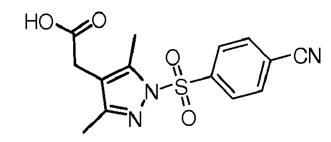

### {1-[(4-Cyanophenyl)sulfonyl]-3,5-dimethyl-1H-pyrazol-4-yl}acetic acid

The title compound of Preparation 5 (70 mg, 0.18 mmol) was treated with trifluoroacetic acid according to the method of Example 1, followed by purification by reverse-phase chromatography using the SP1 Purification System to give 6 mg (0.012 mmol, 6%) of the title compound as an oil. Purity 97%.
¹H NMR (400 MHz, CHLOROFORM-*d*) δ ppm 8.07 (2 H, d, *J*=8.6 Hz), 7.82 (2 H, d, *J*=8.6 Hz), 3.34 (2 H, s), 2.48 (3 H, s), 2.19 (3 H, s).
HPLC/MS (30 min) retention time 11.56 min.
LRMS: *m*/*z* 320 (M+1).

### EXAMPLE 11

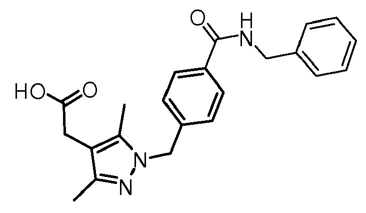

### (1-{4-[(Benzylamino)carbonyl]benzyl}-3,5-dimethyl-1H-pyrazol-4-yl)acetic acid

The title compound of Preparation 24 (49 mg, 0.11 mmol) was treated with trifluoroacetic acid according to the method of Example 1 to give 28 mg (0.07 mmol, 66%) of the title compound as a white solid. Purity 100%.
¹H NMR (400 MHz, DMSO-d6) δ ppm 2.05 (s, 3 H), 2.09 (s, 3 H), 3.27 (s, 2 H), 4.47 (d, J=5.86 Hz, 2 H), 5.25 (s, 2 H), 7.16 (d, J=8.21 Hz, 2 H), 7.20 - 7.28 (m, 1 H), 7.26 - 7.38 (m, 3 H), 7.83 (d, J=8.21 Hz, 2 H), 9.01 (t, J=5.86 Hz, 1 H), 12.16 (br. s., 1 H).
HPLC/MS (30 min) retention time 10.53 min.
LRMS: *m*/*z* 378 (M+1).

### EXAMPLE 12

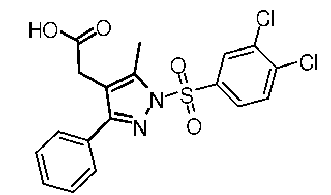

### {1-[(3,4-Dichlorophenyl)sulfonyl]-5-methyl-3-phenyl-1H-pyrazol-4-yl}acetic acid

The title compound of Preparation 19 (20 mg, 0.04 mmol) was treated with trifluoroacetic acid according to the method of Example 1, followed by purification by reverse-phase chromatography using the SP1 Purification System to give 2.4 mg (0.0056 mmol, 14%) of the title compound. Purity 88%.
¹H NMR (partial, 400 MHz, DMSO-d6) δ ppm 8.24 (1 H, d, J=2.0 Hz),, 7.99 (1 H, dd, J=8.5, 2.0 Hz),, 7.95 (0 H, d, J=8.5 Hz),, 7.56 (2 H, d, J=7.0 Hz),, 7.43 - 7.51 (3 H, m),, 3.46 (2 H, br. s.).
HPLC/MS (30 min) retention time 17.51 min.
LRMS: *m*/*z* 426 (M+1).

### EXAMPLE 13

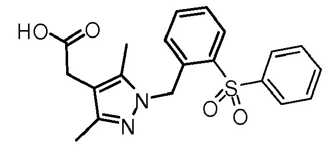

### {3,5-Dimethyl-1-[2-(phenylsulfonyl)benzyl]-1H-pyrazol-4-yl}acetic acid

The title compound of Preparation 28 (120 mg, 0.27 mmol) was treated with trifluoroacetic acid according to the method of Example 1. The residue was treated several times with ether, decanting each time. The residue was dried under reduced pressure to give 64 mg (0.16 mmol, 61 %) of the title compound as a white solid. Purity 99%.
¹H NMR (400 MHz, DMSO-d6) δ ppm 1.73 (s, 3 H), 2.02 (s, 3 H), 3.27 (s, 2 H), 5.36 (s, 2 H), 6.38 (d, J=7.03 Hz, 1 H), 7.58 - 7.71 (m, 4 H), 7.72 - 7.79 (m, 1 H), 7.96 (d, J=7.42 Hz, 2 H), 8.18 (dd, 1 H), 12.19 (s, 1 H).
HPLC/MS (30 min) retention time 11.33 min.
LRMS: *m*/*z* 385 (M+1).

### EXAMPLE 14

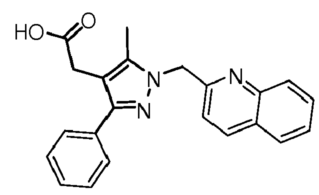

### [5-Methyl-3-phenyl-1-(quinolin-2-ylmethyl)-1H-pyrazol-4-yl]acetic acid

The title compound of Preparation 31 (32 mg, 0.077 mmol) was treated with trifluoroacetic acid according to the method of Example 1. The residue was treated several times with ether, decanting each time. The residue was dried under reduced pressure to give 16 mg (0.045 mmol, 61%) of the title compound as a white solid. Purity 99%.
¹H NMR (400 MHz, DMSO-d6) δ ppm 2.27 (s, 3 H), 3.51 (s, 2 H), 5.64 (s, 2 H), 7.14 (d, J=8.60 Hz, 1 H), 7.30 - 7.38 (m, 1 H), 7.42 (t, J=7.42 Hz, 2 H), 7.55 - 7.68 (m, 3 H), 7.79 (t, J=7.23 Hz, 1 H), 7.99 (dd, J=17.19, 8.21 Hz, 2 H), 8.36 (d, J=8.60 Hz, 1 H), 12.35 (br. s., 1 H).
HPLC/MS (30 min) retention time 16.62 min.
LRMS: *m*/*z* 358 (M+1).

### EXAMPLE 15

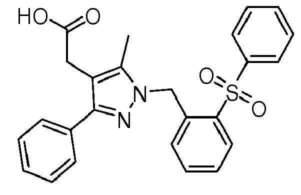

### {5-Methyl-3-phenyl-1-[2-(phenylsulfonyl)benzyl]-1H-pyrazol-4-yl}acetic acid

The title compound of Preparation 32 (95 mg, 0.19 mmol) was treated with trifluoroacetic acid according to the method of Example 1. The residue was treated several times with ether, decanting each time. The residue was dried under reduced pressure to give 46mg (0.10 mmol, 54%) of the title compound as a white solid. Purity 99%.
¹H NMR (400 MHz, DMSO-d6) δ ppm 1.83 (s, 3 H), 3.47 (s, 2 H), 5.53 (s, 2 H), 6.54 (d, J=7.03 Hz, 1 H), 7.29 - 7.36 (m, 1 H), 7.40 (t, J=7.42 Hz, 2 H), 7.53 (d, J=7.42 Hz, 2 H), 7.60 - 7.71 (m, 4 H), 7.72 - 7.80 (m, 1 H), 8.00 (d, J=7.42 Hz, 2 H), 8.21 (dd, 1 H), 12.36 (s, 1 H).
HPLC/MS (15 min) retention time 8.18 min.
LRMS: *m*/*z* 447 (M+1).

### EXAMPLE 16

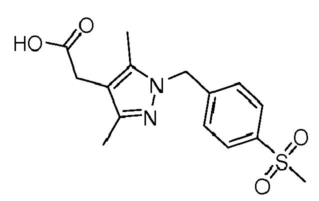

### {3,5-Dimethyl-1-[4-(methylsulfonyl)benzyl]-1H-pyrazol-4-yl}acetic acid

The title compound of Preparation 33 (73 mg, 0.19 mmol) was treated with trifluoroacetic acid according to the method of Example 1. The residue was treated several times with ether, decanting each time. The residue was dried under reduced pressure to give 53 mg (0.16 mmol, 85%) of the title compound as a white solid. Purity 99%.
¹H NMR (400 MHz, DMSO-d6) δ ppm 2.06 (s, 3 H), 2.11 (s, 3 H), 3.19 (s, 3 H), 5.32 (s, 2 H), 7.33 (d, J=8.21 Hz, 2 H), 7.88 (d, J=8.21 Hz, 2 H).
HPLC/MS (30 min) retention time 7.3 min.
LRMS: *m*/*z* 323(M+1).

### EXAMPLE 17

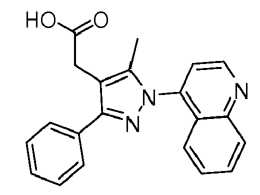

### (5-Methyl-3-phenyl-1-quinolin-4-yl-1H-pyrazol-4-yl)acetic acid

The title compound of Preparation 34 (37 mg, 0.092 mmol) was treated with trifluoroacetic acid according to the method of Example 1. The residue was treated several times with ether, decanting each time. The residue was dried under reduced pressure to give 24 mg (0.069 mmol, 74%) of the title compound as a white solid. Purity 99%.
¹H NMR (400 MHz, DMSO-d6) δ ppm 2.13 (s, 3 H), 3.65 (s, 2 H), 7.39 (t, J=7.23 Hz, 1 H), 7.46 (t, J=7.62 Hz, 3 H), 7.69 (d, J=7.42 Hz, 2 H), 7.78 - 8.01 (m, 2 H), 8.33 (d, J=8.21 Hz, 1 H), 8.70 (br. s., 1 H), 9.53 (br. s., 1 H), 12.46 (br. s., 1 H).
HPLC/MS (30 min) retention time 11.6 min.
LRMS: *m*/*z* 344 (M+1).

### EXAMPLE 18

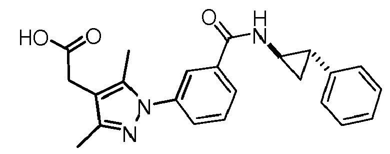

### {3,5-Dimethyl-1-[3-({[trans-2-phenylcyclopropyl]amino}carbonyl)phenyl]-1H-pyrazol-4-yl}acetic acid

The title compound of Preparation 21 (60 mg, 0.13 mmol) was treated with trifluoroacetic acid according to the method of Example 1, followed by purification by reverse-phase chromatography using the SP1 Purification System to give 20 mg (0.051 mmol, 38%) of the title compound as an oil. Purity 98%.
¹H NMR (400 MHz, DMSO-*d*₆) δ ppm 10.46 (1 H, s), 7.85 (1 H, s), 7.52 (1 H, d, *J*=8.2 Hz), 7.40 (1 H, t, *J*=8.0 Hz), 7.30 (2 H, t, *J*=7.4 Hz), 7.17 - 7.24 (3 H, m), 7.15 (1 H, d, *J*=7.8 Hz), 3.37 (2 H, s), 2.35 - 2.43 (1 H, m), 2.24 (3 H, s), 2.14 (3 H, s), 2.03-2.11 (1 H, m), 1.51 (1 H, dt, *J*=9.0, 4.5 Hz), 1.35 - 1.44 (1 H, m).
HPLC/MS (30 min) retention time 13.97 min.
LRMS: *m*/*z* 390 (M+1).

### EXAMPLE 19

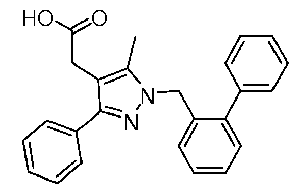

### [1-(Biphenyl-2-ylmethyl)-5-methyl-3-phenyl-1H-pyrazol-4-yl]acetic acid

The title compound of Preparation 35 (100 mg, 0.23 mmol) was treated with trifluoroacetic acid according to the method of Example 1. The residue was treated several times with ether, decanting each time. The residue was dried under reduced pressure to give 89 mg (0.23 mmol, 100%) of the title compound as a white solid. Purity 100%.
¹H NMR (400 MHz, DMSO-d6) δ ppm 1.96 (s, 3 H), 3.44 (s, 2 H), 5.25 (s, 2 H), 6.85 - 6.90 (m, 1 H), 7.26 - 7.46 (m, 9 H), 7.47 - 7.53 (m, 2 H), 7.54 - 7.59 (m, 2 H).
HPLC/MS (30 min) retention time 16.22 min.
LRMS: *m*/*z* 383 (M+1).

### EXAMPLE 20

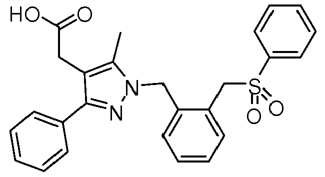

### (5-Methyl-3-phenyl-1-{2-[(phenylsulfonyl)methyl]benzyl}-1H-pyrazol-4-yl)acetic acid

The title compound of Preparation 36 (78 mg, 0.15 mmol) was treated with trifluoroacetic acid according to the method of Example 1. The residue was treated several times with ether, decanting each time. The residue was dried under reduced pressure to give 63 mg (0.13 mmol, 91 %) of the title compound as a white solid. Purity 100%.
¹H NMR (400 MHz, DMSO-d6) δ ppm 2.10 (s, 3 H), 3.47 (s, 2 H), 5.01 (s, 2 H), 5.49 (s, 2 H), 6.74 (d, J=7.82 Hz, 1 H), 7.10 (d, J=7.42 Hz, 1 H), 7.21 (t, J=7.42 Hz, 1 H), 7.26 - 7.35 (m, 2 H), 7.41 (t, J=7.62 Hz, 2 H), 7.57 (d, J=7.42 Hz, 2 H), 7.64 (t, J=7.62 Hz, 2 H), 7.78 (t, J=7.23 Hz, 1 H), 7.85 (d, J=7.42 Hz, 2 H), 12.32 (s, 1 H).
HPLC/MS (30 min) retention time 13.9 min.
LRMS: *m*/*z* 461 (M+1).

### EXAMPLE 21

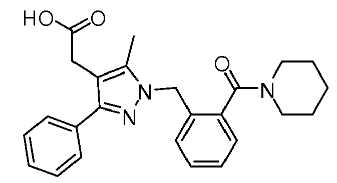

### {5-Methyl-3-phenyl-1-[2-(piperidin-1-ylcarbonyl)benzyl]-1H-pyrazol-4-yl}acetic acid

The title compound of Preparation 40 (17 mg, 0.036 mmol) was treated with trifluoroacetic acid according to the method of Example 1. The residue was treated several times with ether, decanting each time. The residue was dried under reduced pressure to give 16 mg (0.036 mmol, 100%) of the title compound as a white solid. Purity 96%.
¹H NMR (400 MHz, DMSO-d6) δ ppm 1.22 - 1.32 (m, 2 H), 1.50 (br. s., 4 H), 2.19 (s, 3 H), 2.81 (br. s., 1 H), 3.02 (br. s., 1 H), 3.32 (br. s., 1 H), 3.46 (br. s., 2 H), 3.76 (br. s., 1 H), 5.16 (br. s., 1 H), 5.31 (br. s., 1 H), 7.11 (d, J=6.64 Hz, 1 H), 7.23 (d, J=6.64 Hz, 1 H), 7.27 - 7.34 (m, 1 H), 7.34 - 7.43 (m, 4 H), 7.53 (d, J=7.03 Hz, 2 H).
HPLC/MS (30 min) retention time 12.90 min.
LRMS: *m*/*z* 418 (M+1).

### EXAMPLE 22

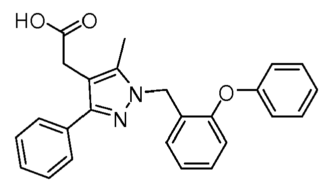

### [5-Methyl-1-(2-phenoxybenzyl)-3-phenyl-1H-pyrazol-4-yl]acetic acid

The title compound of Preparation 41 (123 mg, 0.27 mmol) was treated with trifluoroacetic acid according to the method of Example 1. The residue was treated several times with ether, decanting each time. The residue was dried under reduced pressure to give 55 mg (0.13 mmol, 51 %) of the title compound as a white solid. Purity 100%.
¹H NMR (400 MHz, DMSO-d6) δ ppm 2.19 (s, 3 H), 3.44 (s, 2 H), 5.35 (s, 2 H), 6.88 (d, J=8.21 Hz, 1 H), 6.95 - 7.03 (m, 3 H), 7.09 - 7.18 (m, 2 H), 7.26 - 7.34 (m, 2 H), 7.36 - 7.43 (m, 4 H), 7.54 - 7.58 (m, 2 H), 12.29 (s, 1 H).
HPLC/MS (30 min) retention time 15.98 min.
LRMS: *m*/*z* 399 (M+1).

### EXAMPLE 23

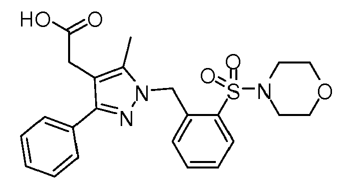

### {5-Methyl-1-[2-(morpholin-4-ylsulfonyl)benzyl]-3-phenyl-1H-pyrazol-4-yl}acetic acid

The title compound of Preparation 42 (177 mg, 0.35 mmol) was treated with trifluoroacetic acid according to the method of Example 1. The residue was treated several times with ether, decanting each time. The residue was dried under reduced pressure to give 114 mg (0.25 mmol, 72%) of the title compound as a white solid. Purity 100%.
¹H NMR (400 MHz, DMSO-d6) δ ppm 2.18 (s, 3 H), 3.04 - 3.12 (m, 4 H), 3.53 (s, 2 H), 3.62 - 3.69 (m, 4 H), 5.70 (s, 2 H), 6.67 (d, J=7.42 Hz, 1 H), 7.31 - 7.37 (m, 1 H), 7.42 (t, J=7.42 Hz, 2 H), 7.52 - 7.62 (m, 3 H), 7.66 (t, J=7.23 Hz, 1 H), 7.90 (d, J=7.82 Hz, 1 H), 12.36 (s, 1 H).
HPLC/MS (30 min) retention time 12.85 min.
LRMS: *m*/*z* 456 (M+1).

### EXAMPLE 24

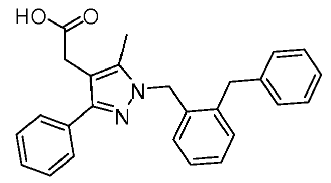

### [1-(2-Benzylbenzyl)-5-methyl-3-phenyl-1H-pyrazol-4-yl]acetic acid

The title compound of Preparation 44 (105 mg, 0.23 mmol) was treated with trifluoroacetic acid according to the method of Example 1. The residue was treated several times with ether, decanting each time. The residue was dried under reduced pressure to give 81 mg (0.20 mmol, 89%) of the title compound as a white solid. Purity 100%.
¹H NMR (400 MHz, DMSO-d6) δ ppm 2.02 (s, 3 H), 3.47 (s, 2 H), 4.16 (s, 2 H), 5.32 (s, 2 H), 6.58 (d, J=7.42 Hz, 1 H), 7.13 - 7.26 (m, 6 H), 7.27 - 7.35 (m, 3 H), 7.40 (t, J=7.62 Hz, 2 H), 7.58 (d, J=7.42 Hz, 2 H), 12.32 (s, 1 H).
HPLC/MS (30 min) retention time 16.68 min.
LRMS: *m*/*z* 397 (M+1).

### EXAMPLE 25

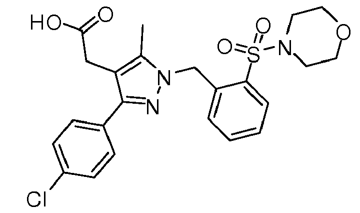

### {3-(4-Chlorophenyl)-5-methyl-1-[2-(morpholin-4-ylsulfonyl)benzyl]-1H-pyrazol-4-yl}acetic acid

The title compound of Preparation 48 (112 mg, 0.20 mmol) was treated with trifluoroacetic acid according to the method of Example 1. The residue was treated several times with ether, decanting each time. The residue was dried under reduced pressure to give 70 mg (0.14 mmol, 70%) of the title compound as a white solid. Purity 99%.
¹H NMR (400 MHz, DMSO-d6) δ ppm 2.17 (s, 3 H), 3.09 (t, 4 H), 3.53 (s, 2 H), 3.64 (t, 4 H), 5.70 (s, 2 H), 6.66 (d, J=7.42 Hz, 1 H), 7.48 (d, J=8.60 Hz, 2 H), 7.52 - 7.72 (m, 4 H), 7.90 (d, J=7.03 Hz, 1 H), 12.38 (br. s., 1 H).
HPLC/MS (30 min) retention time 14.60 min.
LRMS: *m*/*z* 490 (M+1).

### EXAMPLE 26

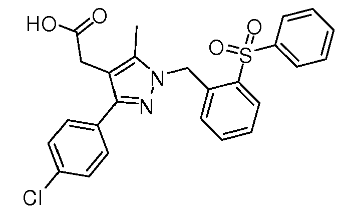

### {3-(4-Chlorophenyl)-5-methyl-1-[2-(phenylsulfonyl)benzyl]-1H-pyrazol-4-yl}acetic acid

The title compound of Preparation 49 (170 mg, 0.32 mmol) was treated with trifluoroacetic acid according to the method of Example 1. The residue was treated several times with ether, decanting each time. The residue was dried under reduced pressure to give 119 mg (0.24 mmol, 78%) of the title compound as a white solid. Purity 100%.
¹H NMR (400 MHz, DMSO-d6) δ ppm 1.85 (s, 3 H), 3.47 (s, 2 H), 5.54 (s, 2 H), 6.57 (d, J=7.03 Hz, 1 H), 7.42 - 7.50 (m, 2 H), 7.52 - 7.58 (m, 2 H), 7.58 - 7.70 (m, 4 H), 7.74 (t, 1 H), 7.98 (d, J=7.42 Hz, 2 H), 8.18 - 8.23 (m, 1 H), 12.35 (br. s., 1 H).
HPLC/MS (30 min) retention time 15.35 min.
LRMS: *m*/*z* 481 (M+1).

### EXAMPLE 27

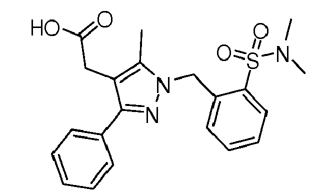

### (1-{2-[(Dimethylamino)sulfonyl]benzyl}-5-methyl-3-phenyl-1H-pyrazol-4-yl)acetic acid

The title compound of Preparation 52 (80 mg, 0.17 mmol) was treated with trifluoroacetic acid according to the method of Example 1. The residue was treated several times with ether, decanting each time. The residue was dried under reduced pressure to give 45 mg (0.11 mmol, 65%) of the title compound as a white solid. Purity 99%.
¹H NMR (400 MHz, DMSO-d6) δ ppm 2.14 (s, 3 H), 2.80 (s, 6 H), 3.52 (s, 2 H), 5.69 (s, 2 H), 6.61 (d, J=7.42 Hz, 1 H), 7.35 (t, 1 H), 7.42 (t, J=7.42 Hz, 2 H), 7.54 (t, J=7.23 Hz, 1 H), 7.57 - 7.66 (m, 3 H), 7.87 (d, J=7.82 Hz, 1 H), 12.34 (br. s., 1 H).
HPLC/MS (30 min) retention time 12.97 min.
LRMS: *m*/*z* 414 (M+1).

### EXAMPLE 28

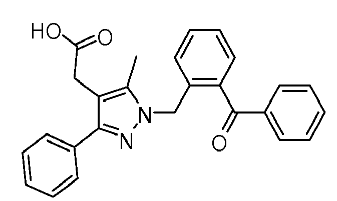

### [1-(2-Benzoylbenzyl)-5-methyl-3-phenyl-1H-pyrazol-4-yl]acetic acid

The title compound of Preparation 54 (16 mg, 0.034 mmol) was treated with trifluoroacetic acid according to the method of Example 1. The residue was treated several times with ether, decanting each time. The residue was dried under reduced pressure to give 6.8 mg (0.012 mmol, 43%) of the title compound as a white solid. Purity 99%.
¹H NMR (400 MHz, CHLOROFORM-d) δ ppm 2.15 (s, 3 H), 3.49 (s, 2 H), 5.53 (s, 2 H), 6.96 (d, J=7.82 Hz, 1 H), 7.28 - 7.47 (m, 8 H), 7.51 - 7.60 (m, 3 H), 7.77 (d, J=7.42 Hz, 2 H).
HPLC/MS (30 min) retention time 14.75 min.
LRMS: *m*/*z* 411 (M+1).

### EXAMPLE 29

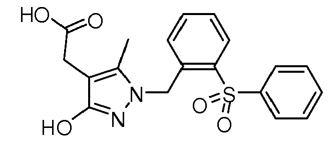

### {3-Hydroxy-5-methyl-1-[2-(phenylsulfonyl)benzyl]-1H-pyrazol-4-yl}acetic acid

The title compound of Preparation 57 (158 mg, 0.36 mmol) was treated with trifluoroacetic acid according to the method of Example 1. The residue was treated several times with ether, decanting each time. The residue was dried under reduced pressure to give 128 mg (0.25 mmol, 72%) of the title compound as a white solid. Purity 100%.
¹H NMR (400 MHz, DMSO-d6) δ ppm 2.07 (s, 3 H), 3.16 (s, 2 H), 5.45 (s, 2 H), 7.58 - 7.65 (m, 3 H), 7.68 - 7.73 (m, 3 H), 7.88 - 7.92 (m, 2 H), 8.12 (d, J=7.81 Hz, 1 H), 11.49 (s, 1 H), 12.12 (s, 1 H).
HPLC/MS (30 min) retention time 10.97 min.
LRMS: *m*/*z* 387 (M+1).

### EXAMPLE 30

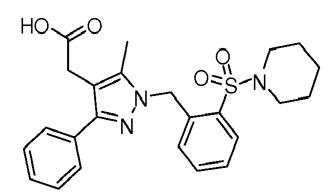

### {5-Methyl-3-phenyl-1-[2-(piperidin-1-ylsulfonyl)benzyl]-1H-pyrazol-4-yl}acetic acid

The title compound of Preparation 60 (28 mg, 0.055 mmol) was treated with trifluoroacetic acid according to the method of Example 1. The residue was treated several times with ether, decanting each time. The residue was dried under reduced pressure to give 24 mg (0.053 mmol, 96%) of the title compound as a white solid. Purity 100%.
¹H NMR (400 MHz, CHLOROFORM-d) δ ppm 1.26 (s, 1 H), 1.60 - 1.70 (m, 4 H), 2.16 (s, 3 H), 3.19 (t, 4 H), 3.62 (s, 2 H), 5.83 (s, 2 H), 6.66 (d, J=7.82 Hz, 1 H), 7.31 - 7.50 (m, 5 H), 7.64 (d, J=7.03 Hz, 2 H), 7.96 (d, J=7.82 Hz, 1 H).
HPLC/MS (30 min) retention time 14.82 min.
LRMS: *m*/*z* 454 (M+1).

### EXAMPLE 31

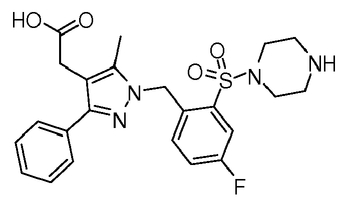

### {1-[4-Fluoro-2-(piperazin-1-ylsulfonyl)benzyl]-5-methyl-3-phenyl-1H-pyrazol-4-yl}acetic acid

The title compound of Preparation 63 (13 mg, 0.025 mmol) was treated with trifluoroacetic acid according to the method of Example 1. The residue was treated several times with ether, decanting each time. The residue was dried under reduced pressure to give 9 mg (0.019 mmol, 92%) of the title compound as a white solid. Purity 100%.
¹H NMR (400 MHz, DMSO-d6) δ ppm 2.18 (s, 3 H), 3.21 (br. s., 4 H), 3.36 (br. s., 4 H), 3.52 (s, 2 H), 5.65 (s, 2 H), 6.81 (d, J=5.08 Hz, 1 H), 7.35 (t, 1 H), 7.42 (t, J=7.62 Hz, 2 H), 7.52 - 7.67 (m, 3 H), 7.74 (d, J=8.60 Hz, 1 H), 8.73 (br. s., 1 H).
HPLC/MS (30 min) retention time 8.85 min.
LRMS: *m*/*z* 473 (M+1).

### PHARMACOLOGICAL ACTIVITY

### CRTh2 radioligand binding assay

The assay is performed using membranes obtained from CHO.K1 cells stably overexpressing the CRTh2 receptor and ³[H]PGD₂ as radioligand. The assay is performed incubating 2-4 µg of membranes per well with the compound to be tested and 5 nM ³[H]PGD₂ in incubation buffer (50 mM HEPES pH 7.0, 10 mM MgCl₂, 1 mM EDTA, 0.2% BSA) for 2 h at room temperature. The reaction is terminated by filtering in GF/C plates pre-treated with 0.3% de polyetilenimine buffer and washing 3 times with wash buffer (50 mM HEPES pH 7.4, 0.5 M NaCl). After washing, plates are dried and scintillation cocktail Optiphase Hisafe II added. The radioactivity retained in the filter is counted using a Microbeta liquid scintillation counter. Compound IC₅₀s are determined using Excel XL-fit for calculations.

### CRTh2 GTPγS antagonism binding assay

The assay is performed using membranes obtained from CHO.K1 cells stably overexpressing the CRTh2 receptor and [³⁵S]-GTPγS as radioligand. Assay is performed pre-incubating 4-8 µg of membranes per well with the compound to be tested for 1 h, followed by incubation with PGD₂ at 50 nM (EC₈₀), 0.1 nM [³⁵S]-GTPγS in incubation buffer (20 mM HEPES, 10 mM MgCl₂, 100 mM NaCl, 10 µM GDP, 10 µg/ml Saponine and 0.2% BSA) for 2 h at room temperature. The reaction is terminated by filtering in GF/C plates pre-treated with 20 mM HEPES, 10 mM MgCl₂, 100 mM NaCl and washing 6 times with wash buffer (20 mM NaH₂PO4, 20 mM Na₂HPO4). After washing, plates are dried and scintillation buffer Optiphase added. The radioactivity retained in the filter is counted using a Microbeta liquid scintillation counter. Compound IC₅₀s are determined using Excel XL-fit for calculations.

### CRTh2 Isolated cell eosinophil cell shape assay

Citrated whole blood is obtained from consenting donors and polymorphonuclear leukocytes obtained by gradient centrifugation on Polymorphprep for 40 min at 500G brake off. PMN fraction is resuspended in calcium free buffer (10 mM HEPES, 10 mM glucose, 0.1 % BSA in calcium free PBS) and centrifuged for 10 min at 400G at room temperature. Lysis of red blood cells is performed by adding 20 ml of 0.2% saline buffer

(0.2% NaCl) for 40 s and stopped by adding 20 ml of 1.6% saline buffer (1.6% NaCl). For compound treatment 90 µl of PMNs cells resuspended at a density of 5 x 10⁶ cells/ml in 10 mM HEPES, 10 mM glucose, 0.1 % BSA in calcium containing PBS are incubated for 10 min with 10 µl of compound to be tested followed by incubation with 10µl of 500 nM PGD₂ for exactly 4 min at 37ºC. Fixation is performed by addition of 0.2 ml of ice-cold 1:5 diluted CellFix (BD Biosciences) and immediately analysed on a FACSCalibur (BD Biosciences). Eosinophils are gated on the basis of autofluorescence in the FL2 channels and shape change of 600 cells is assayed by forward scatter and side scatter analysis. Compound IC₅₀s are determined using Excel XL-fit for calculations.

### CRTh2 whole blood eosinophil cell shape assay

Whole blood is obtained from consenting donors mixed with heparin and kept in rotation until use. For compound treatment, 90 µl of blood are mixed with 10 µl of compound to be tested and incubated for 10 min at room temperature followed by addition of 10 µl of 500 nM PGD₂ for exactly 4 min at 37ºC. Reaction was stopped by placing the tubes on ice and adding of 0.25 ml of ice-cold 1:5 diluted CellFix (BD Biosciences). Red blood cells are lysed in two steps by adding 2 ml of lysis solution (150mM ammonium chloride, 10mM potassium hydrogencarbonate), incubating for 20 and 10 min respectively and centrifuging at 300G for 5 min at 6ºC. The pellet is resuspended in 0.2 ml of fixative solution and immediately analized on a FACSCalibur (BD Biosciences). Eosinophils are gated on basis of autofluorescence in the FL2 channels and shape change of 600 cells is assayed by forward scatter and side scatter analysis. Compound IC₅₀s are determined using Excel XL-fit for calculations

| **Example** | **GTPγ Binding (nM)** |
|---|---|
| 1 | 250 |
| 4 | 370 |
| 13 | 86 |
| 15 | 6,8 |
| 23 | 74 |
| 24 | 105 |
| 25 | 94 |
| 26 | 24 |
| 29 | 160 |
| 30 | 66 |

### COMBINATION PRODUCT

The pyrazole derivatives of the invention may also be combined with other active compounds in the treatment of diseases indicated above. For example the compounds of the present invention can be combined with active substances which are known to be useful in the treatment of these diseases.

Examples of such active substances are (a) anticholinergics, such as Aclidinium Bromide or Tiotropium, (b) corticosteroids, or gluococorticoids such as prednisone or methylprednisolone; (c) antihistamines, such as Ebastine, Ranitidine, ABT-239 or JNJ 7777120, (d) beta-2 agonists, such as salmeterol or formoterol, (e) chemokine receptor antagonists, such as Maraviroc or Enfuvirtide, (f) Leukotriene receptor antagonists, (g) JAK inhibitors such as tasocitinib citrate or INCB018424, (h) Syk inhibitors such as R-112, (i) Phosphosdiesterase IV inhibitors such as GRC-4039, (j) p38 Inhibitors such as ARRY-797; (k) PKC inhibitors such as NVP-AEB071; (I) 5-lipoxygenase activating protein inhibitors, such as Veliflapon; (m) 5-lipoxygenase inhibitors, (n) CYSLTR1 antagonists, such as Montelukast, Pranlukast or Zafirlukast; (o) CYSLTR2 antagonists, such as Pranlukast, Zafirlukast or Tipilukast; (p) BLT1 antagonists, (q) BLT2 antagonists, (r) Thromboxane A2 antagonists such as Ramatroban; (s) DP1 receptor antagonists, such as Laropiprant; (t) DP1 receptor agonists, such as BW-245C; (u) IP receptor agonists, such as RO-1138452; (v) anti-IgE, such as Omalizumab; (w) IL5 antibody, such as Mepolizumab; (x) leukotriene formation inhibitors, (y) bronchodilators, such as pirbuterol, epinephrine, ephedrine, salbutamol or salmeterol; (z) decongestants, such as ephedrine, Levo-methamphetamine, Naphazoline, Oxymetazoline, Phenylephrine, Phenylpropanolamine, Propylhexedrine, Pseudoephedrine, Synephrine or Tetrahydrozoline; (aa) mucolytics such as Acetylcysteine, Ambroxol, Bromhexine, Carbocisteine, Domiodol, Eprazinone, Erdosteine, Letosteine, Neltenexine, Sobrerol, Stepronin or Tiopronin; (bb) antitussives, such as dectromethorphan; (cc) analgesics such as aspirin, paracetamol, rofecoxid, celecoxib, morphine, codeine, oxycodone, hydrocodone, dihydromorphine or flupirtine and (dd) Expectorants such Antimony pentasulfide, Guaiacolsulfonate, Guaifenesin, Potassium iodide or Tyloxapol.

Accordingly, another embodiment of the invention is a combination product comprising (i) at least a compound of formula (I) as defined previously, and (ii) one or more active ingredients as described above, for simultaneous, separate or sequential use in the treatment of the human or animal body.

The combinations of the invention may be used in the treatment of disorders which are susceptible to amelioration by CRTh2 receptor antagonists. Thus, the present application encompasses methods of treatment of these disorders, as well as the use of the combinations of the invention in the manufacture of a medicament for the treatment of these disorders.

Preferred examples of such disorders are asthma, chronic obstructive pulmonary disease (COPD), allergic rhinitis, atopic dermatitis, psoriasis, emphysema, eosinophilic bronchitis, being more preferably asthma, chronic obstructive pulmonary disease (COPD), allergic rhinitis and atopic dermatitis.

The amount of each active which is required to achieve a therapeutic effect will, of course, vary with the particular active, the route of administration, the subject under treatment, and the particular disease or disorder being treated.

The active ingredients may be administered from 1 to 6 times a day, sufficient to exhibit the desired activity. Preferably, the active ingredients are administered once or twice a day, most preferably once a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

Examples of suitable β2-agonists that can be combined with the compounds of formula (I) are terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, formoterol, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in PCT patent applications Nos. WO 2007/124898, WO 2006/122788A1, WO 2008/046598, WO 2008095720, WO 2009/068177 and WO 2010/072354.

Examples of suitable corticosteroids and glucocorticoids that can be combined with the compounds of formula (I) are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, Desisobutyrylciclesonide, hydrocortisone acetate, hydrocortisone sodium succinate, prednisolone sodium phosphate and hydrocortisone probutate, Prednisolone sodium metasulfobenzoate and clobetasol propionate.

Examples of suitable anticholinergics that can be combined with the compounds of formula (I) are tiotropium salts, oxitropium salts, flutropium salts, ipratropium salts, glycopyrronium salts, trospium salts, zamifenacin, revatropate, espatropate, darotropium bromide, Cl-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-histamines that can be combined with the compounds of formula (I) are Methapyrilene, Mequitazine, Azelastine hydrochloride, Ranitinide, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, ABT-239, JNJ7777120, Vapitadine hydrochloride, AZD-1744, GSK-1004723D, GSK-835726, SUN-1334H.

Examples of suitable PDE4 inhibitors that can be combined with the compounds of formula (I) are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, UK-500001, BLX-914, 2-carbomethoxy-4-cyano-4-(3-cyclopropylmethoxy-4-difluroromethoxyphenyl)cyclohexan1-one, *cis* [4-cyano-4-(3-cyclopropylmethoxy-4-difluoromethoxyphenyl)cyclohexan-1-ol, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number WO 03/097613, WO 2004/058729, WO 2005/049581, WO 2005/123693, WO 2005/123692, and WO 2010/069504.

Examples of suitable Syk kinase inhibitors that can be combined with the compounds of formula (I) are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Particularly preferred combination products according to the invention comprise a compound of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, formoterol, salmeterol, indacaterol, carmoterol, LAS 100977, compounds described in PCT patent applications Nos. WO 2008/077639, WO 2009/021696, WO 2009/153043, and WO 2010/083975 (in particular amino(iso)nicotinic acid derivatives selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid and 2-(3,5-difluoro-2-methylbiphenyl-4-ylamino)nicotinic acid; and azabiphenylaminobenzoic acid derivatives selected from the group consisting of 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid and 5-methyl-2-((6-(2,3-difluorophenyl)pyridin-3-yl)amino)benzoic acid), methapyrilene, cetirizine, loratadine, ebastine, desloratadine, fexofenadine, azelastine, levocabastine, olopatadine, Montelukast, picremolimus, tacrolimus, mupiricin, retapamulin, clotrimazole, ketoconazole and terbinafine.

Preferred combination products according to the invention comprises a compound of formula (I) and a therapeutically effective amount of one or more additional therapeutic agents selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, rolipram, roflumilast, cilomilast and the compounds claimed in the PCT patent applications number WO 03/097613, WO 2004/058729, WO 2005/049581, WO 2005/123693, WO 2005/123692, and WO 2010/069504.

Thus, in one aspect of the invention, the combination product comprises a compound of formula (I) and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In another aspect of the invention, the combination product comprises a compound of formula (I) and an anticholinergic agent. Particulary preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]-octane salts. The combination product may further comprise a corticosteroid selected from the group consisting of mometasone furoate, ciclesonide, budesonide, fluticasone furoate and fluticasone propionate.

In a still other aspect of the invention, the combination product comprises a compound of formula (I) and a suitable β2-agonist. Particularly preferred β2-agonists are those selected from the group consisting of formoterol, salmeterol, indacaterol, carmoterol, LAS100977 and compound described in PCT patent applications Nos. WO 2007/124898, WO 2006/122788A1, WO 2008/046598, WO 2008095720, WO 2009/068177 and WO 2010/072354. The composition may further comprise a corticosteroid selected from the group consisting of mometasone furoate, ciclesonide, budesonide, fluticasone furoate, and fluticasone propionate. In addition to the compound of formula (I) and to the β2-agonist, the combination product may further comprise an anticholinergic agent selected from the group consisting of tiotropium salts, glycopyrronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

In a still other aspect of the invention, the combination product comprises a compound of formula (I) and a PDE4 inhibitor. Particularly preferred PDE4 inhibitors are those selected from the group consisting of rolipram, roflumilast, cilomilast and the compounds claimed in the PCT patent applications number WO 03/097613, WO 2004/058729, WO 2005/049581, WO 2005/123693, WO 2005/123692, and WO 2010/069504. The combination product may further comprise a corticosteroid selected from the group consisting of mometasone furoate, ciclesonide, budesonide, fluticasone furoate and fluticasone propionate. In addition to the compound of the invention and to the PDE4 inhibitor, the combination product may further comprise an anticholinergic agent selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

In a preferred embodiment of the present invention, the combination product comprises a compound of formula (I) and a therapeutically effective amount of a 3-[2-Hydroxy-2,2-bis(2-thienyl)acetoxy]-1-(3-phenoxypropyl)-1-azoniabicyclo[2.2.2]octane salts. Optionally, the combination product further comprises a corticosteroid and/or a PDE4 inhibidor.

In another preferred embodiment of the present invention, the combination product comprises a compound of formula (I) and a therapeutically effective amount of a mometasone furoate. Optionally, the combination product further comprises an anticholinergic salt and/or a PDE4 inhibidor.

In yet another embodiment of the invention, the combination product comprises a compound of formula (I), a corticosteroid, an anticholinergic agent and a PDE4 inhibidor.

The active compounds in the combination product may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be administered in the morning and the other (s) later in the day. Or in another scenario, one or two actives could be administered twice daily and the other (s) once daily, either at the same time as one of the twice-a-day dosing occurred, or separately. Preferably at least two, and more preferably all, of the actives would be administered together at the same time. Preferably, at least two, and more preferably all actives would be administered as an admixture.

### PHARMACEUTICAL COMPOSITIONS

Pharmaceutical compositions according to the present invention comprise the compounds of the invention in association with a pharmaceutically acceptable diluent or carrier.

As used herein, the term pharmaceutical composition refers to a mixture of one or more of the compounds described herein, or physiologically/pharmaceutically acceptable salts, solvates, N-oxides, stereoisomers, deuterated derivatives thereof or prodrugs thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

A pharmaceutically acceptable excipient refers to an inert substance added to a pharmaceutical composition to further facilitate administration of a compound.

The invention further provides pharmaceutical compositions comprising the compounds of the invention in association with a pharmaceutically acceptable diluent or carrier together with one or more other therapeutic agents such as the previously described for use in the treatment of a pathological condition or disease susceptible to amelioration by CRTh2 receptor antagonists.

The invention is also directed to pharmaceutical compositions of the invention for use in the treatment of a pathological disease or disorder susceptible to amelioration by CRTh2 receptor antagonists, in particular wherein the pathological disease or disorder is as described above.

The invention also provides a method of treatment of a pathological condition or disease susceptible to amelioration by CRTh2 receptor antagonists in particular wherein the pathological condition or disease described above, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a pharmaceutically acceptable excipient such as a carrier or diluent. The active ingredient may comprise 0.001% to 99% by weight, preferably 0.01% to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21 st Edition, Lippincott Williams & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatin, vegetable oils and polyethylene glycols.

Additional suitable carriers for formulations of the compounds of the present invention can be found in Remington: The Science and Practice of Pharmacy, 21st Edition, Lippincott Williams & Wilkins, Philadelphia, Pa., 2001.

### i) Oral Administration

The compounds of the invention may be administered orally (peroral administration; per os (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y." 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified release. Modified release formulations include delayed-, sustained-, pulsed-, controlled-, targeted and programmed release.

Suitable modified release formulations are described in U.S. Patent No. 6,106,864. Details of other suitable release technologies such as high energy dispersions and osmotic and coated particles can be found in Verma et al, Pharmaceutical Technology On-line, 25(2), 1-14 (2001). The use of chewing gum to achieve controlled release is described in WO 00/35298. The disclosures of these references are incorporated herein by reference in their entireties.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.
Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-50 mg, more preferably 0.01-5 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/000325 and W02006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the respimat® which is described, for example, in PCT Patent Applications Nos. W0 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

### iv) Nasal mucosal administration

The compounds of the invention may also be administered via the nasal mucosal.

Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Preferably the composition is in unit dosage form, for example a tablet, capsule or metered aerosol dose, so that the patient may administer a single dose.

The following preparations forms are cited as formulation examples:

**Formulation Example 1 (Oral suspension)**

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

**Formulation Example 2 (Hard gelatine capsule for oral administration)**

| **Ingredient** | **Amount** |
|---|---|
| Active Compound | 1 mg |
| Lactose | 150 mg |
| Magnesium stearate | 3 mg |

**Formulation Example 3 (Gelatin cartridge for inhalation)**

| **Ingredient** | **Amount** |
|---|---|
| Active Compound (micronized) | 0,2 mg |
| Lactose | 25 mg |

**Formulation Example 4 (Formulation for inhalation with a DPI)**

| **Ingredient** | **Amount** |
|---|---|
| Active Compound (micronized) | 15 mg |
| Lactose | 3000 mg |

**Formulation Example 5 (Formulation for a MDI)**

| **Ingredient** | **Amount** |
|---|---|
| Active Compound (micronized) | 10 g |
| 1,1,1,2,3,3,3-heptafluoro-n-propane | q.s. to 200 ml |

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, IV oxide, solvate, stereoisomer or deuterated derivative thereof: 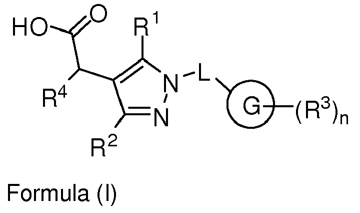 wherein:
• R¹ represents a linear or branched C₁-C₄ alkyl group;
• R² is selected from the group consisting of a linear or branched C₁-C₄ alkyl group, a hydroxyl group, a C₁-C₄ alkoxy group and a C₆₋₁₀ aryl group, wherein the aryl group is optionally substituted by one or more substituents selected from the group consisting of a halogen atom, a C₁-C₄ alkyl group, a C₁-C₄ alkoxy group, a cyano group, a -CF₃ group and a nitro group;
• each R³ is the same or different and is selected from the group consisting of a halogen atom, a cyano group, a carboxy group, a C₆-C₁₀ aryl group, a C₆-C₁₀ aryloxy group, a (C₆-C₁₀ aryl)-(C₁-C₄ alkyl) group, a 3- to 7-membered heterocyclyl group containing at least one heteroatom selected from N, S and O, a (3- to 7-membered heterocyclyl)-(C₁-C₄ alkyl) group, a 5- to 10-membered heteroaryl group containing at least one heteroatom selected from N, S and O, a (5- to 10-membered heteroaryl)-(C₁-C₄ alkyl) group, a -(CH₂)₀₋₁-SO₂R^{a} group, a -O(CH₂)₀₋₁-R^{a} group, a -NHCOR^{a} group, a -CONHR^{a} group, a -NHCONHR^{a} group, a -NHSO₂R^{a} group, a -SO₂N(CH₃)R^{a} group and a -COR^{a} group;
• R⁴ is selected from the group consisting of a hydrogen atom and a linear or branched C₁-C₄ alkyl group;
• R^{a} is selected from the group consisting of a linear or branched C₁-C₄ alkyl group, a C₆-C₁₀ aryl group, a (C₆-C₁₀ aryl)-(C₁-C₄ alkyl) group, a (C₆-C₁₀ aryl)-(C₂-C₆ alkenyl) group, a (C₆-C₁₀ aryl)-(C₃-C₇ cycloalkyl) group, a 3- to 7-membered heterocyclyl group containing at least one heteroatom selected from N, S and O, a 5- to 10-membered heteroaryl group containing at least one heteroatom selected from N, S and O; and a (5- to 10-membered heteroaryl)-(C₁-C₄ alkyl) group, wherein the aryl and heteroaryl groups are optionally substituted by one or more substituents selected from the group consisting of a linear or branched C₁-C₄ alkyl group, a C₁-C₄ alkoxy group, a halogen atom, a - CF₃ group, a -CN group and a -SO₂(C₁-C₄ alkyl) group;
• n has a value of 0 to 3;
• L is selected from the group consisting of a direct bond, a -CR^{b}R^{c}- group and a - SO₂- group;
• R^{b} and R^{c} are independently selected from the group consisting of a hydrogen atom and a linear or branched C₁-C₄ alkyl group; or R^{b} and R^{c} together with the carbon atom to which they are attached form a C₃-C₇ cycloalkyl group;
• G is selected from the group consisting of a phenyl group, a pyridyl group, a quinolinyl group and an isoquinolinyl group;
with the proviso that when G is phenyl or pyridyl group, n can not have a value of 0; with the additional proviso that when L is a direct bond or a methylene group, R₃ is other than a halogen atom or a -CN group;
and with the additional proviso that the compound is not 2-(3,5-dimethyl-1-(4-(piperidine-1-carbonyl)phenyl)-1H-pyrazol-4-yl)acetic acid or 2-(1-(biphenyl-4-yl)-3,5-dimethyl-1H-pyrazol-4-yl)acetic acid.

2. A compound according to claim 1 wherein R¹ is selected from the group consisting of a linear C₁-C₂ alkyl group, and preferably R¹ represents a methyl group.

3. A compound according to claim 1 or 2, wherein R² is selected from the group consisting of a C₁-C₂ alkyl group, a hydroxyl group and a phenyl group, wherein the phenyl group is optionally substituted with a halogen atom, preferably R² is selected from the group consisting of a methyl group, a hydroxy group and a phenyl group, wherein the phenyl group is optionally substituted with a chlorine atom.

4. A compound according to any one of claims 1 to 3, wherein R⁴ is selected from the group consisting of a hydrogen atom and a C₁-C₂ alkyl group, and preferably R⁴ represents a hydrogen atom.

5. A compound according to any one of claims 1 to 4, wherein L is selected from the group consisting of a -CH₂- group and a -SO₂-group.

6. A compound according to any one of claims 1 to 5, wherein G represents a phenyl group.

7. A compound according to any one of claims 1 to 6, wherein R³ is selected from the group consisting of a benzyl group, a -(CH₂)₀₋₁-SO₂R^{a} group, a -NHCOR^{a} group and a -NHSO₂R^{a} group, wherein R^{a} is selected from the group consisting of a C₆-C₁₀ aryl group and a 3- to 7-membered heterocyclyl group, wherein the aryl group is optionally substituted by one or two substituents selected from the group consisting of a linear or branched C₁-C₄ alkyl group, a C₁-C₄ alkoxy group, a halogen atom, a - CF₃ group, a -CN group and a -SO₂(C₁-C₄ alkyl) group and n is 0 or 1.

8. A compound according to claim 7, wherein R³ represents a -SO₂R^{a} group, wherein R^{a} is selected from the group consisting of a phenyl group and a morpholinyl group and n has a value of 1.

9. A compound according to any one of claims 1 to 8, wherein R¹ represents a methyl group, R² is selected from the group consisting of a methyl group, a hydroxy group and a phenyl group, wherein the phenyl group is optionally substituted with a chlorine atom, R⁴ represents a hydrogen atom, L is selected from the group consisting of a -CH₂- group and a -SO₂-group, G represents a phenyl group, R³ is selected from the group consisting of a -SO₂R^{a} group, wherein R^{a} is selected from the group consisting of a phenyl group and a morpholinyl group and n has a value of 1.

10. A compound according to claim 1, wherein
• R¹ is selected from the group consisting of a methyl group;
• R² is selected from the group consisting of a methyl group, a hydroxyl group and a phenyl group optionally substituted by a chlorine atom;
• R³ is selected from the group consisting of a chlorine atom, a cyano group, a carboxy group, a phenyl group, a phenoxy group, a benzyl group, a -(CH₂)₀₋₁-SO₂R^{a} group, a -O(CH₂)₀₋₁-R^{a} group, a -NHCOR^{a} group, a -CONHR^{a} group, a -NHCONHR^{a} group, a -NHSO₂R^{a} group, a -SO₂N(CH₃)R^{a} group and a -COR^{a} group;
• R⁴ represents a hydrogen atom;
• R^{a} is selected from the group consisting of a methyl group, a phenyl, a benzyl group, a phenyl-(C₂-C₆ alkenyl) group, a phenyl-cyclopropyl group, a piperidin-1-yl group, a morpholinyl group, a piperazin-1-yl group, wherein the phenyl group is optionally substituted by one or two substituents selected from the group consisting of a methyl group, a chlorine atom, a fluorine atom and a -CF₃ group,
• n has a value of 0, 1 or 2;
• L is selected from the group consisting of a direct bond, a -CH₂- group and a - SO₂-group; and
• G is selected from the group consisting of a phenyl group and a quinoline group.

11. A compound according to claim 1 which is one of:
{3,5-dimethyl-1-[3-({[trans-2-phenylcyclopropyl]carbonyl}amino)phenyl]-1H-pyrazol-4-yl}acetic acid ;
[3,5-dimethyl-1-(3-{[4-(trifluoromethyl)benzoyl]amino}benzyl)-1H-pyrazol-4-yl]acetic acid ;
[3,5-dimethyl-1-(3-{[(4-methylphenyl)sulfonyl]amino}benzyl)-1H-pyrazol-4-yl]acetic acid;
{3,5-dimethyl-1-[3-({[(4-methylphenyl)amino]carbonyl}amino)benzyl]-1H-pyrazol-4-yl}acetic acid;
(1-{3-[(3-fluorobenzoyl)amino]benzyl}-3,5-dimethyl-1H-pyrazol-4-yl)acetic acid;
[1-(3-{[(2,5-dichlorophenyl)sulfonyl]amino}benzyl)-3,5-dimethyl-1H-pyrazol-4-yl]acetic acid;
[3,5-dimethyl-1-(3-{[(4-methylphenyl)sulfonyl]amino}phenyl)-1H-pyrazol-4-yl]acetic acid;
(1-{3-[(benzylsulfonyl)amino]phenyl}-3,5-dimethyl-1H-pyrazol-4-yl)acetic acid;
{1-[3-({[(4-fluorophenyl)amino]carbonyl}amino)phenyl]-3,5-dimethyl-1H-pyrazol-4-yl}acetic acid;
{1-[(4-cyanophenyl)sulfonyl]-3,5-dimethyl-1H-pyrazol-4-yl}acetic acid;
(1-{4-[(benzylamino)carbonyl]benzyl}-3,5-dimethyl-1H-pyrazol-4-yl)acetic acid;
{1-[(3,4-dichlorophenyl)sulfonyl]-5-methyl-3-phenyl-1H-pyrazol-4-yl}acetic acid;
{3,5-dimethyl-1-[2-(phenylsulfonyl)benzyl]-1H-pyrazol-4-yl}acetic acid;
[5-methyl-3-phenyl-1-(quinolin-2-ylmethyl)-1H-pyrazol-4-yl]acetic acid;
{5-methyl-3-phenyl-1-[2-(phenylsulfonyl)benzyl]-1H-pyrazol-4-yl}acetic acid;
{3,5-dimethyl-1-[4-(methylsulfonyl)benzyl]-1H-pyrazol-4-yl}acetic acid;
(5-methyl-3-phenyl-1-quinolin-4-yl-1H-pyrazol-4-yl)acetic acid;
{3,5-dimethyl-1-[3-({[trans-2-phenylcyclopropyl]amino}carbonyl)phenyl]-1H-pyrazol-4-yl}acetic acid;
[1-(biphenyl-2-ylmethyl)-5-methyl-3-phenyl-1H-pyrazol-4-yl]acetic acid;
(5-methyl-3-phenyl-1-{2-[(phenylsulfonyl)methyl]benzyl}-1H-pyrazol-4-yl)acetic acid;
{5-methyl-3-phenyl-1-[2-(piperidin-1-ylcarbonyl)benzyl]-1H-pyrazol-4-yl}acetic acid;
[5-methyl-1-(2-phenoxybenzyl)-3-phenyl-1H-pyrazol-4-yl]acetic acid;
{5-methyl-1-[2-(morpholin-4-ylsulfonyl)benzyl]-3-phenyl-1H-pyrazol-4-yl}acetic acid;
[1-(2-benzylbenzyl)-5-methyl-3-phenyl-1H-pyrazol-4-yl]acetic acid;
{3-(4-chlorophenyl)-5-methyl-1-[2-(morpholin-4-ylsulfonyl)benzyl]-1H-pyrazol-4-yl}acetic acid;
{3-(4-chlorophenyl)-5-methyl-1-[2-(phenylsulfonyl)benzyl]-1H-pyrazol-4-yl}acetic acid;
(1-{2-[(dimethylamino)sulfonyl]benzyl}-5-methyl-3-phenyl-1H-pyrazol-4-yl)acetic acid;
[1-(2-benzoylbenzyl)-5-methyl-3-phenyl-1H-pyrazol-4-yl]acetic acid;
{3-hydroxy-5-methyl-1-[2-(phenylsulfonyl)benzyl]-1H-pyrazol-4-yl}acetic acid;
{5-methyl-3-phenyl-1-[2-(piperidin-1-ylsulfonyl)benzyl]-1H-pyrazol-4-yl}acetic acid,
and
{1-[4-fluoro-2-(piperazin-1-ylsulfonyl)benzyl]-5-methyl-3-phenyl-1H-pyrazol-4-yl}acetic acid
or a pharmaceutically acceptable salt or N-oxide or solvate, or stereoisomers deuterated derivative thereof.

12. A compound according to any one of claims 1 to 11 for use in the treatment of the human or animal body by therapy.

13. A compound according to any one of claims 1 to 11 for use in the treatment of a pathological condition or disease mediated by CRTh2 antagonists which condition or disease is preferably selected from asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, Crohn's disease, ulcerative colitis, irritable bowel disease and inflammatory bowel disease, more preferably selected from asthma, chronic obstructive pulmonary disease (COPD), allergic rhinitis and atopic dermatitis.

14. A pharmaceutical composition comprising a compound as defined in any one of claims 1 to 11 in association with a pharmaceutically acceptable diluent or carrier.

15. A method for treating a subject afflicted with a pathological condition or disease as defined in claim 13, which comprises administering to said subject an effective amount of a compound as defined in any one of claims 1 to 11.

16. A combination product comprising (i) a compound according to any one of claims 1 to 11; and (ii) another compound selected from
a. anticholinergics, such as Aclidinium Bromide or Tiotropium
b. corticosteroids, or gluococorticoids such as prednisone or methylprednisolone
c. antihistamines, such as Ebastine, Ranitidine, ABT-239 or JNJ 7777120
d. beta-2 agonists, such as salmeterol or formoterol
e. chemokine receptor antagonists, such as Maraviroc or Enfuvirtide
f. leukotriene receptor antagonists,
g. JAK inhibitors such as tasocitinib citrate or INCB018424
h. Syk inhibitors such as R-112,
i. Phosphosdiesterase IV inhibitors such as GRC-4039
j. p38 Inhibitors such as ARRY-797
k. PKC inhibitors such as NVP-AEB071
1. 5-lipoxygenase activating protein inhibitors, such as Veliflapon
m. 5-lipoxygenase inhibitors,
n. CYSLTR1 antagonists, such as Montelukast, Pranlukast or Zafirlukast
o. CYSLTR2 antagonists, such as Pranlukast, Zafirlukast or Tipilukast
p. BLT1 antagonists,
q. BLT2 antagonists,
r. Thromboxane A2 antagonists such as Ramatroban.
s. DP1 receptor antagonists, such as Laropiprant
t. DP1 receptor agonists, such as BW-245C
u. IP receptor agonists, such as RO-1138452
v. anti-IgE, such as Omalizumab
w. IL5 antibody, such as Mepolizumab
x. leukotriene formation inhibitors,
y. bronchodilators, such as pirbuterol, epinephrine, ephedrine or salbutamol
z. decongestants, such as ephedrine, Levo-methamphetamine, Naphazoline, Oxymetazoline, Phenylephrine, Phenylpropanolamine, Propylhexedrine, Pseudoephedrine, Synephrine or Tetrahydrozoline aa
. mucolytics such as Acetylcysteine, Ambroxol, Bromhexine, Carbocisteine, Domiodol, Eprazinone, Erdosteine, Letosteine, Neltenexine, Sobrerol, Stepronin or Tiopronin
bb. antitussives, such as dectromethorphan
cc. analgesics such as aspirin, paracetamol, rofecoxid, celecoxib, morphine, codeine, oxycodone, hydrocodone, dihydromorphine or flupirtine and
dd. Expectorants such Antimony pentasulfide, Guaiacolsulfonate, Guaifenesin, Potassium iodide or Tyloxapol,
for simultaneous, separate or sequential use in the treatment of the human or animal body.
